# EUROPÄISCHE PATENTSCHRIFT

(11) **EP 1 250 040 B1**
(45) Veröffentlichungstag und Bekanntmachung des Hinweises auf die Patenterteilung: **05.04.2006**
(21) Anmeldenummer: 01907400.4
(22) Anmeldetag: 03.01.2001
(51) Int. Cl.: A01M 1/20, A61L 9/03

(54) **WIRKSTOFFCHIP MIT INTEGRIERTEM HEIZELEMENT**
CHIP THAT COMPRISES AN ACTIVE AGENT AND AN INTEGRATED HEATING ELEMENT
PUCE A MATIERE ACTIVE ET A ELEMENT CHAUFFANT INTEGRE

(30) Priorität: 13.01.2000 DE 10001035
(43) Veröffentlichungstag der Anmeldung: 23.10.2002
(73) Patentinhaber: S.C.JOHNSON & SON, INC., Racine, WI 53403-2236 (US)
(72) Erfinder: NEUMANN, Hermann, 42781 Haan (DE); KALDER, Dietmar, 40764 Langenfeld (DE); STEINEL, Heinrich, W., 86825 Bad Wörishofen (DE)
(74) Vertreter: Ruschke, Hans Edvard
(86) Internationale Anmeldenummer: PCT/EP2001/000013
(87) Internationale Veröffentlichungsnummer: WO 2001/050849

(56) Entgegenhaltungen:
- DE-A- 19 605 581
- DE-A- 19 731 156
- FR-A- 2 292 430
- FR-A- 2 322 546
- GB-A- 2 153 227

## Beschreibung

Die Erfindung betrifft einen Wirkstoffchip mit integriertem Heizelement zur Verdampfung von Wirkstoffen aus dem Wirkstoffchip. Sie betrifft insbesondere einen Wirkstoffchips mit mindestens einem integriertem Heizelement, bestehend aus einem leitfähigen, maschinell verarbeitbaren Material und zwei elektrischen Kontakten, aus dem der Wirkstoff durch Aufheizen des Chips durch Anlegen einer elektrischen Spannung an die elektrischen Kontakte verdampft werden kann. Die Erfindung betrifft ferner ein Verfahren zur Herstellung eines solchen Wirkstoffchips mit integriertem Heizelement.

Es sind bereits verschiedene Vorrichtungen zur Verdampfung von Wirkstoffen wie Insektiziden oder Duftstoffen bekannt..

Eine für diesen Zweck geeignete Vorrichtung ist ein Plättchenverdampfer, bestehend aus einem Heizgerät und lnsektizidplättchen. Die Insektizidplättchen bestehen aus Materialien wie Zellstoff- oder Baümwoflkarton, Asbest oder Keramik und sind mit Pyrethroid-Insektiziden imprägniert. Die Insektizidplättchen werden auf das Heizgerät gelegt, das typischerweise eine Temperatur im Bereich von 120 bis 190°C erzeugen kann. Die Insektizide werden durch die Wärme des Heizgerätes aus den Plättchen verdampft. Die Wirkungsdauer bei Plättchenverdampfern ist wegen der hohen Arbeitstemperatur und der ungleichmäßigen Wirkstoffabgabe auf etwa 12 Stunden begrenzt. (FR-A-7 535 835 und FR-A-7 535.836).

Ein ähnliches Prinzip liegt einerri Gelverdampfer zugrunde, in dem Insektizide in eine Gelformulierung eingearbeitet sind (DE 197 31 156 A1).

Eine andere Möglichkeit zur Verdampfung von Wirkstoffen besteht in der Verwen dung von sogenannten Flüssigverdampfern, in denen eine Ftüssigformulierung des Wirkstoffs über ein Dochtsystem durch Erwärmung kontinuierlich verdampft wird (GB 2 153 227).

Polymere Wirkstoffträger, in die insektizide Wirkstoffe eingearbeitet sind, sind aus DE 196 05 581 A1 bekannt. Diese polymeren Wirkstoffträger haben theoretisch eine Arbeitstemperatur von 60 bis 150°C. In der Praxis hat sich jedoch gezeigt, dass eine kontinuierliche Abgaberate des Wirkstoffes über eine Zeitdauer von bis zu 60 Tagen in biologisch wirksamer Menge nur mit einer für die Anwendung unpraktikabel großen Oberfläche oder mit einer Temperatur im Bereich von 140 bis 150°C zu realisieren ist. Zur Verdampfung des Wirkstoffs werden die polymeren Wirkstoffträger auf ein Heizgerät, wie es für Plättchen- und Gelverdampfer bereits bekannt ist, gelegt. Tests haben ergeben, dass im Bereich von 110 bis 100°C die Abgaberate des Wirkstoffes und damit die biologische Wirksamkeit stark abnimmt.

Alle bekannten Verdampfersysteme haben den Nachteil, dass sie ein externes Heizgerät benötigen, um die notwendige Wärme zur Verdampfung des Wirkstoffes zu erzeugen. Ein solches Heizgerät verursacht zusätzliche Kosten und benötigt eine gewissen Platz am Ort der Anwendung. Außerdem lässt es Raum fier Fehlbedienungen. Handelt es sich z.B. um ein regelbares Heizgerät, so kann eine falsche Temperatureinstetlung zu einer Über- oder Unterdosierung des Wirkstoffes führen. Handelt es sich nicht um ein regelbares Heizgerät, so müssen für die Verdampfung von Wirkstoffen mit unterschiedlicher Verdampfungstemperatur unterschiedliche Heizgeräte angeschafft werden.

Nachteilig an den bekannten Verdampfersystemen ist weiterhin der durch das Heizgerät bedingte geringe Wirkungsgrad. Der Wärmeübergang zwischen dem Heizgerät und dem Wirkstoffträger ist schlecht, da kein vollständiger Kontakt zwischen den Oberflächen von Wirkstoffträger und Heizplatte erreicht wird und sich eine isolierende Luftschicht zwischen Teilen des Wirkstoffträgers und dem Heizgerät bilden kann (vgl. FR-A-7 535 835 und FR-A-7 535 836). Dies führt dazu, dass es lange dauert, bis der Wirkstoffträger so stark erwärmt ist, dass der Wirkstoff verdampft. Es werden hohe Temperaturen des Heizgerätes benötigt, um den Wirkstoff in einer Menge zu verdampfen, die z.B. für die effektive Bekämpfung von Insekten notwendig ist. Diese hohen Temperaturen liegen auch am Gehäuse an, so dass Verbrennungsgefahr für den Anwender besteht. Bei hohen Temperaturen besteht, auch die Gefahr, dass andere Teile der Wirkstoffformulierung als der Wirkstoff selber verdampfen und zu einer unnötigen Umweitbeiastung beitragen.

Der schlechte Wärmeübergang führt weiterhin zu einer unvollständigen Austreibung des Wirkstoffs aus dem Wirkstoffträger. Bei polymeren Wirkstoffträgern, die auf einer Heizplatte ausgeheizt wurden, wurde ein Verbleib von bis zu 20 % an Wirkstoff in dem Wirkstoffträger gemessen.

Aufgrund der schlechten Wärmeleitfähgkeit der Polymeren und eventuell auftretender Verformungen der Wirkstoffträger ist keine exakte Temperaturkontrolle möglich, so dass es zu einem ungleichmäßigen Wirkstoffaustritt kommen kann.

Die bekannten Systeme haben den weiteren Nachteil, dass insbesondere die nicht beheizten Flächen des Heizgeräts zum Teil so kühl sind, dass der freigesetzte Wirkstoff gleich wieder an ihnen kondensiert.

Aufgabe der Erfindung war es daher, eine Vorrichtung zur Verdampfung von Wirkstoffen zu finden, die ohne ein externes Heizgerät auskommt und dadurch nicht die mit einem externen Heizgerät verbundenen Nachteile aufweist.

Die Lösung der erfindungsgemäßen Aufgabe besteht in einem Wirkstoffchip, der einen bei Raumtemperatur gebundenen Wirkstoff enthält, wobei sich zumindestens teilweise im Inneren des Chips mindestens ein Heizelement befindet und das Heizelement einen elektrischen Widerstand und elektrische Kontakte aufweist. Das Heizelement ist durch das Anlegen einer elektrischen Spannung an die elektrischen Kontakte aufheizbar und der Wirkstoff daraus verdampfbar.

Die Temperaturkontrolle bei vorgegebenem Heizelement erfolgt über die angelegte Spannung U in Verbindung mit dem Widerstand R des Heizelements. Da die gesamte Heizleistung P des Heizelements in die Erwärmung des Wirkstoffchips umgesetzt wird, ist eine genaue Kontrolle des Wirkstoffaustritts möglich. Die Menge des austretenden Wirkstoffs steigt mit der Temperatur des Heizelements. Die lokale Verteilung des Wirkstoffaustritts hängt von der Wärmeleitfähigkeit des Wirkstoffchips und der Geometrie von Heizelement und Chip ab.

Gegenstand der vorliegenden Erfindung ist gemäß ihrem ersten Aspekt ein Wirkstoffchip (1) mit mindestens einem integrierten Heizelement (2), bestehend aus einem leitfähigen, maschinell verarbeitbaren Material und zwei elektrischen Kontakten (3, 4), aus dem der Wirkstoff durch Aufheizen des Chips (1) durch Anlegen einer elektrischen Spannung an die elektrischen Kontakte (3, 4) verdampft werden kann, wobei der Wirkstoffchip (1) dadurch gekennzeichnet ist, dass das Heizelement (2) in den Wirkstoff oder ein Wirkstoffgemisch, gegebenenfalls in Kombination mit einem Wirkstoffträger, so eingebettet ist, dass das Ganze eine Einheit bildet.

Bevorzugte Ausgestaltungen des erfindungsgemäßen Wirkstoffchips mit dem vorgenannten Aufbau sind - jeweils einzeln oder in Kombination - folgende:
Der Wirkstoffchip bildet vorzugsweise eine rechteckige Platte und weist eine Länge im Bereich von 10 bis 100 mm, eine Breite im Bereich von 5 bis 100 mm und eine Dicke im Bereich von 3 bis 20 mm auf;
in dem Wirkstoffchip ist das Heizelement vorzugsweise seiner Länge nach von dem den Wirkstoff enthaltenden Chipteil umschlossen, sodass sich die Form des Heizelements auf der äußeren Form des Wirkstoffchips abbildet;
in dem Wirkstoffchip liegt der Wirkstoff oder das Wirkstoffgemisch vorzugsweise in flüssiger oder in fester Form vor und ist mit einer für den Wirkstoff oder das Wirkstoffgemisch in flüssiger und fester Form undurchdringbaren und in gasförmiger Form durchdringbaren Oberflächenschicht versehen;
in dem Wirkstoffchip ist der Wirkstoff oder das Wirkstoffgemisch vorzugsweise flüssig, gelförmig oder fest und liegt in einem Gehäuse vor und das Heizelement ist in ihm eingebettet;
in dem Wirkstoffchip ist der Wirkstoff oder das Wirkstoffgemisch vorzugsweise an einen Wirkstoffträger gebunden;
in dem Wirkstoffchip ist der Wirkstoffträger vorzugsweise ein Polymer;
der Wirkstoffchip besteht vorzugsweise im Wesentlichen aus dem Heizelement und dem diesen umgebenden Wirkstoffträger mit Wirkstoff oder Wirkstoffgemisch;
in dem Wirkstoffchip besteht der Wirkstoffträger vorzugsweise aus Kunststoffmaterialien, wie Polyethylen, Polypropylen, TPX®-Typen, Desmopan® 8410, Vestamid® 1800, BAK® 402-005, und der Wirkstoff besteht vorzugsweise aus Transfluthrin®.
in dem Wirkstoffchip besteht das Heizelement vorzugsweise aus Keramik, einem Heilzleiter, einer bedampften Folie oder einem leitfähigen Kunststoff;
in dem Wirkstoffchip besteht das Heizelement vorzugsweise aus einem Heizwiderstand oder PTC;
in dem Wirkstoffchip hat das Heizelement vorzugsweise einen Widerstand von 10 kΩ bis 100 kΩ;
in dem Wirkstoffchip hat das Heizelement vorzugsweise einen Widerstand von 2 kΩ bis 30 kΩ;
die Heizleistung des Heizelements in dem Wirkstoffchip liegt vorzugsweise zwischen 0,1 W und 5 W;
in dem Wirkstoffchip kann mit dem Heizelement vorzugsweise eine Temperatur in dem Bereich von 60 °C bis 140 °C eingestellt werden;
in dem Wirkstoffchip ist das Heizelement (2) vorzugsweise in Form eines Mäanders mit mindestens einem Bogen (21) angeordnet, wobei sich die beiden elektrischen Kontakte (3, 4) an den beiden Enden des Mäanders befinden;
in dem Wirkstoffchip ist das Heizelement (22) vorzugsweise in Form von zwei Mäandern mit mindestens einem Bogen (21) angeordnet, wobei sich jeweils ein elektrischer Kontakt (3,3'; 4,4') an jedem Ende der beiden Mäander befindet;
wobei in dem Wirkstoffchip des vorgenannten Typs die einander zugewandten elektrischen Kontakte (3',4') der beiden Mäander vorzugsweise elektrisch leitend miteinander verbunden sind;
in dem Wirkstoffchip hat das Heizelement (23) vorzugsweise eine Gitter- oder Wabenstruktur;
wobei Heizelement (23) mit der Gitter- oder Wabenstruktur vorzugsweise in zwei Streifen (11, 12) ausgeführt ist, die jeweils an einem Ende kontaktiert und an dem der Kontaktierung entgegengesetzten Ende elektrisch leitend miteinander verbunden sind;
in dem Wirkstoffchip bestehen die elektrischen Kontakte vorzugsweise aus Messingblech oder Kupfer;
der Wirkstoffchip weist vorzugsweise eine in den Chip integrierte Betriebsanzeige (15) auf;
wobei die Betriebsanzeige (15) vorzugsweise eine bipolare LED ist;
In den Wirkstoffchip ist zwischen die Stromversorgung und den Kontakt vorzugsweise ein an sich bekannter Timer-Chip eingesetzt und in den Wirkstoffchip ist vorzugsweise ein zusätzlicher Widerstand integriert;
der Wirkstoffchip enthält vorzugsweise als Wirkstoffe Pyrethroide, acarizide Wirkstoffe, Duftstoffe oder ätherische Öle;
wobei er als Wirkstoff vorzugsweise Transfluthrin® oder Pynamin forte® oder Benzylbenzoat enthält;
aus dem Wirkstoffchip kann der Wirkstoff oder das Wirkstoffgemisch vorzugsweise bei einer Temperatur von unter 100 °C, besonders bevorzugt im Bereich von 65 °C bis 90 °C, verdampft werden;
der Wirkstoffchip hat vorzugsweise einen solchen Aufbau, dass nach einer Anfangsphase von 5 Zyklen ä 8 Stunden die abgedampfte Menge an Gesamtformulierung im Mittel über einen Zyklus mindestens 70 %, bevorzugt mehr als 90 %, besonders bevorzugt mehr als 99 % Wirkstoff oder Wirkstoffgemisch enthält.

Das Heizelement kann aus einem leitfähigen, maschinell verarbeitbarem Material wie, Keramik, Heizleiter (Heizdraht), bedampfte Folie oder leitfälligem Kunststoff bestehen.

Das Heizelement kann auch aus einem Heizwiderstand oder einem z.B. aus der einer Publikation der Siemens Matushita Components GmbH u. Co KG (Best. Nr. bei Siemens: B 425-P2562 oder im Internet unter www.siemens.com\pr\index.htm, S. 19 bis 40) bekannten Widerstand mit einem positiven Temperatur-Koeffizient (PTC oder auch Kaltleiter genannt) bestehen. Ein PTC besteht beispielsweise aus einer Mischung von Bariumcarbonat, Titanoxid und weiteren Materialien.

Der elektrische Widerstand des Heizelements liegt vorzugsweise zwischen 10 kΩ und 100 kΩ bei 230 V Versorgungsspannung und zwischen 2 kΩ und 30 kΩ bei 110 V Versorgungsspannung. Der elektrische Widerstand R=p*1/A wird einerseits durch die Auswahl des Materials für das Heizelement (Spezifischer Widerstand p) bzw. der Menge an elektrisch leitfähigem Material im dem Grundmaterial wie Keramik oder Kunststoff, andererseits durch die Materialstärke A und die Länge 1 des Heizelements bestimmt. Die Heizleistung P des Heizelements liegt vorzugsweise zwischen 0,1 W und 5 W und ist abhängig von der Betriebsspannung U nach P=U²/R. Mit einer Heizleistung von 0,1 W bis 5 W können bevorzugt Temperaturen im Bereich von 60°C bis 140°C eingestellt werden.

Das Heizelement kann beliebige Formen annehmen, die je nach der gewünschten Wärmeverteilung und Oberfläche des Heizelements ausgewählt werden.

In einer Ausführungsform hat das Heizelement die Form eines Mäanders mit mindestens einem Bogen, wobei sich die beiden elektrischen Kontakte an den beiden Enden des Mäanders befinden.

Bevorzugt hat das Heizelement die Form von zwei Mäandern mit mindestens einem Bogen, wobei sich jeweils ein elektrischer Kontakt an jedem Ende der beiden Mäander befindet und die einander zugewandten elektrischen Kontakte der beiden Mäander elektrisch leitend miteinander verbunden werden können. Diese Ausführungsform hat den besonderen Vorteil, dass entweder beide Mäander getrennt oder gemeinsam kontaktiert werden können. Damit besteht die Möglichkeit, die Heizleistung P=U₁²/R₁ + U₂²/R₂ auch bei unterschiedlichen Spannungen konstant zu hatten.

Die Mäanderform des Heizelements gewährleistet eine gleichmäßige Wärmeverteilung im Chip.

In anderen Ausführungsformen hat das Heizelement die Struktur eines Gitters oder von Waben. Gitter oder Waben gewährleisten ebenfalls eine gleichmäßige Wärmeverteilung im Chip. In einer speziellen Ausführungsform kann das Heizelement mit der Gitter- oder Wabenstruktur in zwei Streifen ausgeführt sein, die jeweils an einem Ende kontaktiert und an dem der Kontaktierung entgegengesetzten Ende elektrisch leitfähig miteinander verbunden sind.

Die elektrischen Kontakte bestehen bevorzugt aus Messingblech oder aus Kupfer.

Die geometrische Form des Wirkstoffchips mit integriertem Heizelement hängt von dem Anwendungsgebiet und vom Herstellungsverfahren ab.

Im einfachsten Fall bildet der Wirkstoffchip eine rechteckige Platte mit elektrischen Kontakten in Verlängerung der Platte an einer der Seiten. Enthält der Wirkstoffchip z.B. einen Wirkstoff zur Bekämpfung von Kakerlaken, so kann es notwendig sein, den Wirkstoffchip in Bodennähe hinter Schränken anzubringen, so dass er flach und möglichst nah an Wand oder Boden aufliegt. In diesem Fall können die elektrischen Anschlüsse senkrecht zur Plattenfläche angeordnet sein.

Der Wirkstoffchip mit integriertem Heizelement in Form einer rechteckigen Platte weist vorzugsweise eine Länge im Bereich von 10 bis 100 mm, eine Breite im Bereich von 5 bis 100 mm und eine Dicke im Bereich von 3 bis 20 mm auf.

Es können auch mehrere Heizelerüente in einen Wirkstoffchip integriert sein, so dass nacheinander verschiedene Bereiche des Wirkstoffchips, die jeweils einem Heizelement zugeordnet sind, ausgeheizt werden können.

In einer anderen Ausführungsform ist das Heizelement nicht in einen flächigen Chip eingebettet, sondern das Heizelement wird seiner Länge nach von dem den Wirkstoff enthaltenden Chipteit umschlossen, so dass sich die Form des Heizelements auf die äußere Form des Wirkstoffchips abbildet.

Über geeignet geformte elektrischen Kontakte kann das Heizelement direkt an eine Stromversorgung angeschlossen werden. Es kann aber auch durch einen geeigneten Adapter, bestehend aus einem Halter und dem Netzanschluss, an eine Stromversorgung angeschlossen werden. Ein und derselbe Adapter kann für Wirkstoffchips mit unterschiedlichen Wirkstoffen und damit unterschiedlichen Verdampfungstemperaturen eingesetzt werden. Er kann noch weitere Aufgaben haben, wie z.B. das Auffangen von Spannungsschwankungen oder die Bereitstellung von Zusatzfunktionen.

Der Wirkstoff kann rein oder als Wirkstoffmischung in flüssiger, gesförmiger oder in fester Form vorliegen. Dabei ist der Wirkstoffchip mit einer für den Wirkstoff oder die Wirkstoffmischung in flüssiger, gelförmiger oder fester Form undurchdringbaren und in gasförmiger Form durchdringbaren Oberflächenschicht versehen. Sobald der Wirkstoff oder die Wirkstoffmischung infolge der Temperaturerhöhung durch das Heizgerät gasförmig wird, kann er die Oberflächenschicht durchdringen.

Der flüssige oder gelförmige oder feste Wirkstoff oder die Wirkstoffmischung liegt in einem Gehäuse vor und das Heizelement ist in ihn eingebettet.

Alternativ kann der Wirkstoff auch an einen Wirkstoffträger gebunden sein, vorzugsweise ist dieser Wirkstoffträger ein Polymer. Besonders bevorzugt besteht der Wirkstoffchip in diesem Fall im Wesentlichen aus dem Heizelement und dem diesen umgebenden Wirkstoffträger mit Wirkstoff.

Der Wirkstoffträger einer bevorzugten Ausführungsform besteht aus Mischungen, die wenigstens einen Wirkstoff und wenigstens ein Polymer mit einem Kristallitschmelzbereich zwischen 100 und 300°C, vorzugsweise zwischen 150 und 250°C, besonders bevorzugt zwischen 150 und 200°C, enthalten. Der Erweichungsbereich wird bei amorphen thermoplastischen Polymeren durch die Glastemperatur und bei teilkristallinen Polymeren durch die Schmelztemperatur belegt. Außerdem können in den Mischungen als weitere Zusätze organische oder anorganische Hilfsstoffe wie Stabilisatoren oder Farbstoffe eingearbeitet sein.

Als Wirkstoffe können in allen Ausführungsformen der Wirkstoffträger insektizide Wirkstoffe wie Pyrethroide, acarizide Wirkstoffe, Duftstoffe oder ätherische Öle verwendet werden. Bevorzugt wird Transfluthrin als Wirkstoff verwendet. Transfluthrin entfaltet eine insektizide Wirkung gegen Mücken, Fliegen und Kakerlaken.

Als pyrethroide Wirkstoffe werden bevorzugt verwendet:
1) Naturpyrethrum,
2) 3-Allyl-2-methyl-cyclopent-2-en-4l-on-1-yl-d/l-cis/trans-chrysanthemat (Allethrin / Pynamin®),
3) 3-Allyl-2-methyl-cyclopent-2-en-4-on-1-yl-d-cis/trans-chrysanthemat (Pynamin forte),
4) d-3-Allyl-2-methyl-cyclopent-2-en-4-on-1-yl-d-trans-chrysanthemat (Exrin®),
5) 3-Allyl-2-methyl-cyclopent-2-en-4-on-1-yl-d-trans-chrysanthemat (Bioallethrin®),
6) N-(3,4,5 ,6-Tetrahydrophthalimido)-methyl-dl-cis/trans-chrysanthemat (Phthalthrin, Neopynamin®),
7) 5-Benzyl-3-furylmethyl-d-cis/trans-chrysanthemat (Resmethrin, Chrysron forte®),
8) 5-(2-Propargyl)-3-furylmethyl-chrysanthemat (Furamethrin®),
9) 3-Phenoxybenzyl-2,2-dimethyl-3-(2,2-dichlorvinyl)-cyclopropancarboxylat (Permethrin, Exmin®),
10) Phenoxybenzyl-d-cis/trans-ctirysanthemat (Phenothrin, Sumithrin®),
11) Cyanophenoxybenzylisopropyl-4-chlorphenylacetat (Fenvalerat, Sumicidin®),
12) (S)-Cyano-3-phenoxybenzyl-(1R,cis)-3-(2,2-dichlorvinyl)-2,2-dimethylcyclopopancarboxylat,
13) (R,S)-Cyano-3-phenoxybenzyl-(1R,1S)-cis/trans-3-(2,2-dichlorvinyl)-2,2-dimethylcyclopopancarboxylat,
14) Cyano-3-phenoxybenzyl-d-cis/trans-chrysanthemat,
15) 1-Ethinyl-2-methyl-2-pentenyl-cis/trans-chrsyanthemat,
16) 1-Ethinyl-2-methyl-2-penteriyl-2,2-dimethyt-3-(2-methyl-1-propenyl)-cyclopropan-1-carboxylat,
17) 1-Ethinyl-2-methyl-2-pentenyl-2,2,3,3-tetramethylcyclopropancarboxylat,
18) 1-Ethinyl-2-methyl-2-pentenyl-2,2-dimethyl-3-(2,2-dichlorvinyl)-cyclopropan-1-carboxylat,
19) 2.3,5,6-Tetraftuorbenzyt-(+)-:1 R-trans-2,2-dimethyl-3-(2,2-dichlorvinyl)-cyclopropancarboxylat (Transfluthrin, Bayothrin®),
oder Mischungen dieser Wirkstoffe.

Besonders bevorzugt werden die Wirkstoffe 3-Allyl-2-methyl-cyclopent-2-en-4-on-1-yl-d-cis/trans-chrysanthemat (Pynamin forte®) und
2,3,5,6-Tetrafluorbenzyl-(+)-1R-trans-2,2-dimethyl-3-(2,2-dichlorvinyl)-cyclopropancarboxylat (Transfluthrin) verwendet.

Als acarizider Wirkstoff wird bevorzugt Benzylbenzoat verwendet.

Geeignete Duftstoffe sind natürliche Duftstoffe wie z.B. Moschus, Zibet, Ambra, Castereum und ähnliche Duftstoffe: Ajowaöl, Mandelöl, Ambrettesamen absol., Angelikawurzelöl, Anisöl, Bäsillkumöl, Lorbeeröl, Benzoinresinoid, Bergarnottessenz, Birkenöl, Rosenholzöl, Pfriernenkraut absol., Cajeputöl, Canangaöl, Gapiscumöl, Kümmelöl, Cardamonöl, Möhrensamenöl, Cassiaöl, Zedernholzöl, Selleriesamenöl, Zimtrindenöl, Zitronenöl, Muskatellersalbeiöl, Nelkenöl, Kognaköl, Korianderöl, Cubebenöl, Kampferöl, Dillöl, Estragonöl. Eukalyptusöl, Fenchelöl süß, Calbanumresinoid, Knoblauchöl, Geraniumöl, Ingweröl, Grapefruitöl, Hopfenöl, Hyacinthe absol., Jasmin absol., Wacholderbeerenöl, Labdanumresinoid, Lavandelöl, Lorbeerblätteröl, Zitronenöl, Lemonengrasöl, Liebstöckelöl, Macisöl, Mandarinenöl, Misoma absol., Myrrhe absol., Senföl, Narcisse absol., Neroliöl, Muskatnüssöl, Eichenmoos absol., Olibanumresinoid, Zwiebelöl, Opoponaxresinoid, Orangenöl, Orangenblütenöl, Iris konkret, Pfefferöl, Pfefferminzöl, Perubalsam, Petitgrainöl, Fichtennadelöl, Rose absol., Rosenöl, Rosmarinöl, Sandelholzöl, Salbeiöl, Krauseminzöl, Styraxöl, Thymianöl, Tolubalsam, Tonkabohnen absol., Tuberose absol., Terpentinöl, Vanilleschoten absol., Vetiveröl, Veilchenblätter absol., Ylang-Ylang-Öl und ähnliche Pflanzenöle.

Geeignet sind auch synthetische Duftstoffe wie Pinen, Limonen und ähnliche Kohlenwasserstoffe; 3,3,5-Trimethylcyclohexanol, Linalool, Geraniol, Nerol, Citronellol, Menthol, Borneol, Borneylmethoxycyclohexanol, Benzylalkohol, Anisalkohol, Zimtalkohol, β-Phenylethylalkohol, cis-3-Hexanol, Terpineol und ähnliche Alkohole; Anethole, Moschusxylol, Isoeugenol, Methyleugenol und ähnliche Phenole; Amylzimtaldehyd, Anisaldehyd, n-Butyraldehyd, Cuminaldehyd, Cyclamenaldehyd, Decylaldehyd, lsobutyraldehyd, Hexylaldehyd, Heptylaldehyd, n-Nonylaldehydnonadienol, Citral, Citronellal, Hydroxycitronellal, Benzaldehyd, Methylnonylacetaldehyd, Zimtaldehyd, Dodecanol, Hexylzimtaldehyd, Undecanal, Heliotropin, Vahillin, Ethylvanillin und ähnliche Aldehyde, Methylamylketon, Methyl-β-naphthylketon, Methylnonylketon, Moschusketon, Diacetyl, Acetylpropionyl, Acetylbutyryl, Carvon, Methon, Campher, Acetophenon, p-Methylacetophenon, Jonon, Methylionon und ähnliche Ketone; Amylbutyrolacton, Diphenyloxid, Methylphenylglycidat, Nonylaceton, Cumarin, Cineol, Ethylmethylphenylglycidat und ähnliche Lactone bzw. Oxide, Methylformiat, lsopropylformiat, Linalylformiat, Ethylacetat, Octylacetat, Methylacetat, Benzylacetat, Cinnamylacetat, Butylpropionat, lsoamylacetat, lsopropylisobutyrat, Geranylisovalerat, Allylcapronat, Butylheptylat, Octylcaprylat, Methylheptincarboxylat, Methyloctincarboxylat, Isoamylcaprylat, Methyllaurat, Ethylmyristat, Methylmyristat, Ethylbenzoat, Methylcarbinylphenylacetat, Isobutylphenylacetat, Methylcinnamat, Styracin, Methylsalicylat, Ethylanisat, Methylanthranilat, Ethylpyruvat, Ethyl-butylbutyrat, Benzylpropionat, Butylacetat, Butylbutyrat, p-tert.-Butylcyelohexylacetat, Cedrylacetat, Citronellylacetat, Citronellylformiat, p-Cresylacetat, Ethylbutyrat, Ethylcaproat, Ethylcinnamat, Ethylphenylacetat, Ethylenbrassylat, Geranylacetat, Geranylformiat, Isoamyisalicylat, Isoamylvalerat, Isobornylacetat, Linalylacetat, Methylanthranilat, Methyldihydrojasmonat, Nonylacetat, β-Phenylethylacetat, Trichlormethylenphenylcarbinylacetat, Terpinylacetat, Vetiverylacetat und ähnliche Ester. Diese Duftstoffe können einzeln verwendet werden oder mindestens zwei davon können im Gemisch miteinander verwendet werden. Neben dem Duftstoff kann die Formulierung gegebenenfalls zusätzlich die in der Duftstoffindustrie üblichen Zusatzstoffe, wie Patchouliöl bzw. ähnliche flüchtigkeitshemmende Mittel, wie Eugenol bzw. ähnliche viskositätsreguiierende Mittel enthalten.

Als polymere Materialien für den Wirkstoffträger werden bevorzugt amorphe und teilkristalline Polymere sowie Mischungen aus beiden verwendet, die sich thermoplastisch, d.h. als zähflüssige Schmelze verarbeiten lassen und deren Erweichungsbereich unterhalb des Siedepunktes der einzuarbeitenden Wirkstoffe unter Normaldruck liegt. Die. Polymeren werden für den entsprechenden Wirkstoff so gewählt, dass sich der Wirkstoff zumindest teilweise mit den Polymeren mischt.

Als geeignete amorphe Polymere werden bevorzugt verwendet:

PVC (WEICH), Polystyrol, Styrol/Bufadien, Styrol/Acrylnitril, Acrylnirtril/Butadien/Styrol, Polymethylacrylat, amorphe Polycycloolefine, Celluloseester, aromatische Polycarbonate, amorphe aromatische Polyamide, Polyphenylenether, Poly(ether)-sulfone, Polyimide. ,

Als geeignete teilkristalline Polymere werden bevorzugt verwendet:

Polyethylen, Polypropylen, Polybutylern, PVC (HART), Polyamid, Polyetheramide, Polyesteramide, Polyoxymethyfen, Poly-4-methylpenten-1, Polyethylenterephthalat, Polybutylen-terephthälat, Polyimid, Polyether(ether)keton und Polyurethane.

Bevorzugte Mischungen sind beispielsweise: Blends aus Polycarbonaten mit Polybutylenterephthalat, Blends aus Polyamid-6 und Styrol/Acrylnitril.

Besonders bevorzugt sind Polypropyle:n, amorphe aromatische Polyamide, aromatische Polycarbonate und aromatische Polyurethane und ®TPX -Typen, ®Desmopan 8410, ®Vestamid 1800, ®BAK 402-005.

Die Mischungen können mit Hilfe von Antioxidantien stabilisiert werden, indem man einen UV-Absorber als Additiv der Formulierung beimischt. Als UV-Absorber können alle bekannten UV-Absorber eingesetzt werden.

Bevorzugt eingesetzt werden Phenolderivate, wie z.B. Butylhydroxytoluol (BHT), Butylhydroxyanisol (BHA), Bisphenolderivate, Arylamine, wie z.B. Phenylnaphthylamin, Phenyl-β-naphthylamin, ein Kondensat aus Phenetidin und Aceton o.ä. oder Benzophenone.

Es können Farbstoffe, wie anorganische Pigmente, z.B. Eisenoxid, Titanoxid, Ferrocyanblau, und organische Farbstoffe, wie z.B. Alizarin, Azo- und Metallphthalocyanin-Farbstoffe, und Metaitsatze, wie Salze von Eisen, Mangan, Bor, Kupfer, Kobalt, Molybdän und Zink, verwendet werden.

Über die Wirkstoffkonzentration und -menge kann die Wirkdauer in einem Zeitraum von 1 bis 60 Nächten ä 10 Stünden eingestellt werden.

Die Wirkstoffchips enthalten im allgemeinen zwischen 0,1 und 80 Gew.-%, vorzugsweise zwischen 0,2 und 40 Gew.-%, besonders bevorzugt zwischen 1,0 und 20 Gew.% Wirkstoff.

Es können Zusatzfunktionen entweder in den Chip oder in den Adapter integriert werden.

Der Wirkstoffchip kann zusätzlich mit einer Betriebsanzeige ausgestattet sein. Diese kann in einer LED, bevorzugt einer bipolaren LED, bestehen und z.B. mit dem Heizelement in Reihe geschattet werden.

Zwischen die Stromversorgung und die Kontakte des Wirkstoffchips kann ein an sich bekannter Timer-Chip eingesetzt werden. Zusätzlich zu den vorhandenen Kontakten wird dann in den Wirkstoffchip oder in den Adapter ein Widerstand integriert. Dieser Widerstand soll bevorzugt asymmetrisch angeordent sein, so dass der Wirkstoffchip nur in einer von zwei möglichen Einbaupositionen an der Stromversorgung bzw. dem Timer-Chip kontaktiert ist. Der eingebaute Widerstand wird durch die am Timer-Chip eingestellte Timerfunktion so angesteuert, dass nach Ende einer vorausgewählten Zeit der Stromkontakt abbricht. Der Benutzer hat durch einfaches Drehen des Chips die Möglichkeit, zwischen zwei Betriebsarten (mit Timerlohne Timer) zu wählen. Alternativ wäre auch die Wahl zwischen zwei verschiedenen Timerzeiten denkbar.

Zur Stabilisierung der Temperatur bei Schwankungen der Umgebungstemperatur kann ein Widerstand mit einem positiven Temperatur-Koeffizient in das Gerät eingebaut werden. Sinkt die Umgebungstemperatur und damit die Temperatur im Chip ab, so verringert sich die an dem PTC anliegende Temperatur. Durch die Verringerung der Temperatur im PTC verringert sich auch der Widerstand des PTC und der PTC heizt zum Ausgleich.

Gegenstand der Erfindung ist gemäß ihrem zweiten Aspekt ein Verfahren zur Herstellung eines Wirkstoffchips, wie er vorstehend beschrieben worden ist, das dadurch gekennzeichnet ist, dass mindestens ein Heizelement (23) an ein Endlosmetallband angespritzt wird, die einzelnen Heizelemente (23) aus dem Endlosmetallband ausgestanzt, die Kontakte (13, 14) getrennt und die Heizelemente (23) in ein Gehäuse eingelegt werden, der Wirkstoff oder das Wirkstoffgemisch (34) zu dem mindestens einen Heizelement (23) in das Gehäuse (33, 35) gegeben und das Gehäuse geschlossen wird.

Bevorzugte Ausgestaltungen des erfindungsgemäßen Verfahrens zur Herstellung eines Wirkstoffchips sind, einzeln oder in Kombination, die folgenden:
mindestens ein Heizelement (23) wird an ein Endlosmetallband angespritzt, die einzelnen Heizelemente (23) werden aus dem Endlosmetallband ausgestanzt, die Kontakte (13, 14) werden getrennt und in ein den Wirkstoff oder das Wirkstoffgemisch (34) enthaltendes Gehäuse (33, 35) eingelegt und das Gehäuse wird geschlossen;
zur Herstellung eines Wirkstoffchips mit einem Heizelement (23), das aus zwei Streifen (13, 14) besteht, wird vorzugsweise jeder Streifen (13, 14) an zwei Endlosmetallbänder (31, 32) angespritzt, das eine Endlosmetallband (31) zwischen den zwei Streifen wird durchtrennt, das andere Endlosmetallband (32) wird zwischen jedem Streifen durchtrennt und die auf diese Weise hergestellten Heizelemente (23) werden in ein Gehäuse (33, 35), das den Wirkstoff oder das Wirkstoffgemisch (34) enthält, eingelegt und das Gehäuse wird geschlossen;
zur Herstellung eines Wirkstoffchips mit einem Heizelement (23), der aus zwei Streifen (13, 14) besteht, wird vorzugsweise das erste Metallband (31) zwischen jedem Streifen durchtrennt und zwischen je zwei Streifen wird eine LED (15) eingelötet;
zur Herstellung eines Wirkstoffchips, bei dem der Wirkstoff an einen Wirkstoffträger gebunden ist, wird vorzugsweise ein Heizelement (23) an mindestens ein Endlosmetallband angespritzt der Wirkstoffträger wird um das Heizelement am Endlosmetallband herum gespritzt und dann werden die einzelnen Wirkstoffchips aus dem Band von Wirkstoffträgern am Endlosmetallband ausgestanzt und die Kontakte werden getrennt;
in dem Verfahren zur Herstellung eines Wirkstoffchips, bei dem der Wirkstoff an einen Wirkstoffträger gebunden ist, ist der Wirkstoffträger vorzugsweise ein Polymer und das Heizelement ist vorzugsweise ein leitfähiger Kunststoff und das Heizelement wird vorzugsweise zusammen mit dem Wirkstoffträger extrudiert, so dass ein Kunststoffdraht erhalten wird, dessen Kern das Heizelement bildet und dessen Mantel der Wirkstoffträger bildet.

Zur Herstellung eines Wirkstoffchips, bei dem der Wirkstoff oder die Wirkstoffmischung in flüssiger, gefförmiger oder fester Form vorliegt, wird ein Heizelement an ein Endlosmetallband angespritzt. Anschließend werden die einzelnen Heizelemente aus dem Endlosmetallband ausgestanzt, die Kontakte werden getrennt und die Heizelemente in ein Gehäuse eingelegt. Der Wirkstoff oder die Wirkstoffmischung wird zu dem Heizelement in das Gehäuse gegeben, sofern das Gehäuse den Wirkstoff oder die Wirkstoffmischung noch nicht enthält, und das Gehäuse wird geschlossen.

Ähnlich kann ein Wirkstoffchip mit einem Heizelement bestehend aus zwei Streifen, hergestellt werden. Jeder Streifen wird an zwei Endlosmetallbänder angespritzt. Das eine Endlosmetallbänd wird zwischen je zwei Streifen durchtrennt, das andere Endlosmetallband zwischen jedem Streifen. Die auf diese Weise hergestellten Heizelemente werden in ein Gehäuse, das den Wirkstoffträger enthält, eingelegt. Zur Integration einer Betriebsanzeige kann auch das erste Metallband zwischen jedem Streifen durchtrennt werden. Zwischen je zwei Streifen wird dann eine LED eingelötet.

Zur Herstellung eines Wirkstoffchips, bei dem der Wirkstoff an einen Wirkstoffträger gebunden ist, wird ein Heizelement an mindestens ein Endlosmetallband angespritzt. Anschließend wird der Wirkstoffträger mit dem Wirkstoff um das Heizelement am Endlosmetallband gespritzt und dann werden die einzelnen Wirkstoffchips aus dem Band von Wirkstoffträgern am Endlosmetallband ausgestanzt.

Zur Herstellung eine Wirkstoffchips, bei dem der Wirkstoff an einen Wirkstoffträger gebunden ist und der Wirkstoffträger ein Polymer und das Heizelement ein leitfähiger Kunststoff ist, kann das Heizelement zusammen mit dem Wirkstoffträger extrudiert werden in der Form, dass z.B. ein Kunststoffdraht hergestellt wird, dessen Kern das Heizelement bildet und dessen Mantel der Wirkstoffträger bildet. Dieser Kunststoffdraht kann jede beliebige Form, z.B. eine Mäanderform, annehmen.

In einer weiteren Ausführungsform wird ein Heizelement aus Streifen mit Gitter oder Wabenstruktur an mindestens ein Endlosmetallband angespritzt. Anschließend werden die einzelnen Heizelemente aus dem Endlosmetallband ausgestanzt und eventuell die Kontakte getrennt und der Wirkstoff oder die Wirkstoffmischung wird in die Waben- oder Gitterstruktur des Heizelements eingerakelt und zwar in flüssiger, wachsartiger oder thermoplastischer Form. Zum Einrakeln wird der Wirkstoff oder die Wirkstoffmischung in die Zwischenräume der Gitter- oder Wabenstruktur verteilt. Danach wird das Heizelement in das Gehäuse eingelegt.

Der große Vorteil des erfindungsgemäßen Wirkstoffchips ist der, dass anders als bei allen bekannten Systemen kein externes Heizgerät notwendig ist. Die jeweils für den bestimmten Wirkstoff spezifische Arbeitstemperatur wird im Wirkstoffchip mit integriertem Heizgerät selber erzeugt. Eine Über- oder Unterdosierung durch den Anwender durch die Verwendung einer falschen Heizleistung ist ausgeschlossen. Die Anwendung wird damit einfacher, billiger und gefahrloser.

Der erfindungsgemäße Wirkstoffchip weist vorteilhaft einen hohen Wirkungsgrad bei der Verdampfung des Wirkstoffs auf, da die Wärme durch den Widerstandsheizer im Inneren des Wirkstoffträgers erzeugt und ohne Verluste für die Verdampfung des Wirkstoffs genutzt wird. Die Heizenergie wird also effizient genutzt. Das bedeutet auch, dass die Heiztemperaturen für Wirkstoffe mit niedrigen Verdampfungstemperaturen sehr niedrig bei unter 90°C liegen können ohne Einbußen in der Ausnutzung des Wirkstoffs im Vergleich zu konventionellen Systemen, bei denen eine vergleichbare Wirkstoffmenge erst bei Heiztemperaturen über 100°C freigesetzt wird. Mit dem erfindungsgemäßen Wirkstoffchip ist die Gefahr des Verbrennens des Anwenders beim Umgang mit der Vorrichtung reduziert und die Verdampfung von anderen Bestandteilen der Wirkstofformulierung als dem Wirkstoff selbst kann auch auf Null reduziert werden. Der freigesetzte Wirkstoff wird besser ausgenutzt, da keine Kondensation am Heizgerät stattfindet.

Die niedrige Arbeitstemperatur ermöglicht gleichzeitig den Einsatz von temperaturlabilen Wirkstoffen, die bei Temperaturen von über 100 bis 120°C nicht mehr eingesetzt werden können. Genauso können wegen der niedrigeren Arbeitstemperatur niedrigschmelzende Kunststoffe wie Polypropylen und Polyethylen eingesetzt werden.

Durch die im wesentlichen vollständige Austreibung des Wirkstoffs bleibt kein Rückstand in dem Wirkstoffträger, was die Entsorgung erleichtert, insbesondere, wenn zusätzlich biologisch abbaubare Polymere verwendet werden.

Die Abgabe des Wirkstoffs kann kontrolliert erfolgen da die Oberfläche und die Temperaturverteilung genau definiert und kontrolliert werden können, im Gegensatz zu den Systemen mit den externen Heizgeräten.

Der erfindungsgemäße Wirkstoffchip ist flexibel in der Anwendung, da beliebige Formen verwendet werden können und keine Festlegung des benötigten Platzes durch ein Heizgerät besteht. Sollte ein Adapter verwendet werden, so kann dieser für verschiedene Wirkstoffchips mit integriertem Heizelement mit unterschiedlichen Wirkstoffen verwendet werden.

Der erfindungsgemäße Wirkstoffchip kann zur Bekämpfung von Insekten wie Mücken, Fliegen oder Kakerlaken verwendet werden. Er kann auch für die Verdampfung von Duftstoffen oder ätherischen Ölen, z.B. in Bädern oder Toilettenräumen, verwendet werden.

Die Erfindung wird in den beiliegenden Zeichnungen und in den nachstehend beschriebenen Beispielen näher erläutert, ohne jedoch darauf beschränkt zu sein.

In den beiliegenden Zeichnungen zeigen:
- Fig. 1: eine perspektivische Ansicht eines Kunststoff-Plättchens mit integriertem Heizelement.
- Fig. 2 a-e: schematische Darstellungen von möglichen Formgebungen für das Heizelement:
a. Mäanderform mit einem Mäander und zwei Kontakten.
b. Mäanderform mit zwei Mäandern und je zwei Kontakten.
c. Mäanderform mit zwei Mäandern, die elektrisch leitend verbunden sind.
d. Mäanderform mit zwei Mäandern, die getrennt kontaktiert werden.
e. Gitterform in zwei Streifen mit LED.
- Fig. 3 a-g: die Schritte eines Verfahrens zur Herstellung eines Wirkstoffchips mit integriertem Heizelement in Gitterform und Leuchtdiode.
- Fig. 4: die Anteile der abgedampften Stoffe im Mittelwert über 45 Zyklen.

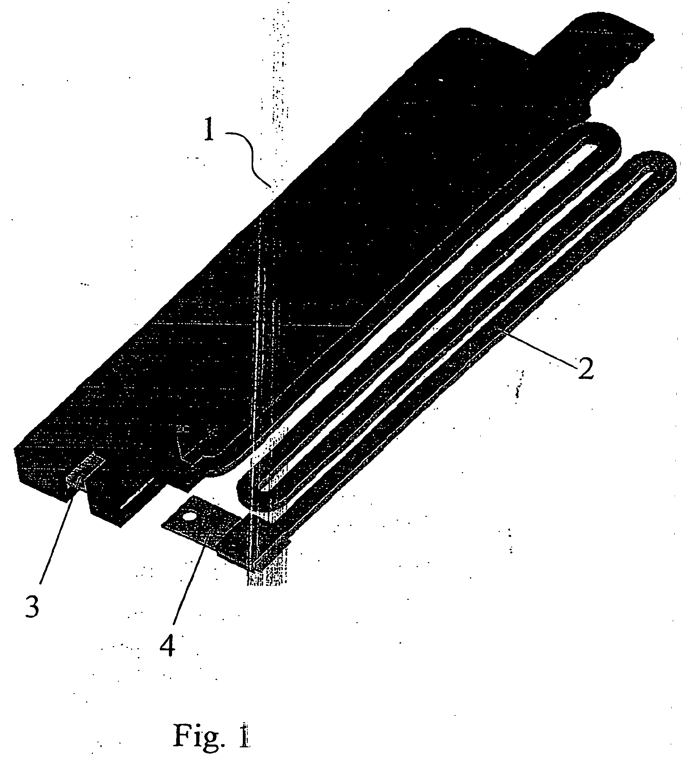

Fig. 1 zeigt eine perspektivische Ansicht eines Wirkstoffchips 1 mit integriertem Heizelement 2. Das Heizelemente 2 hat die elektrischen Kontakte 3 und 4.

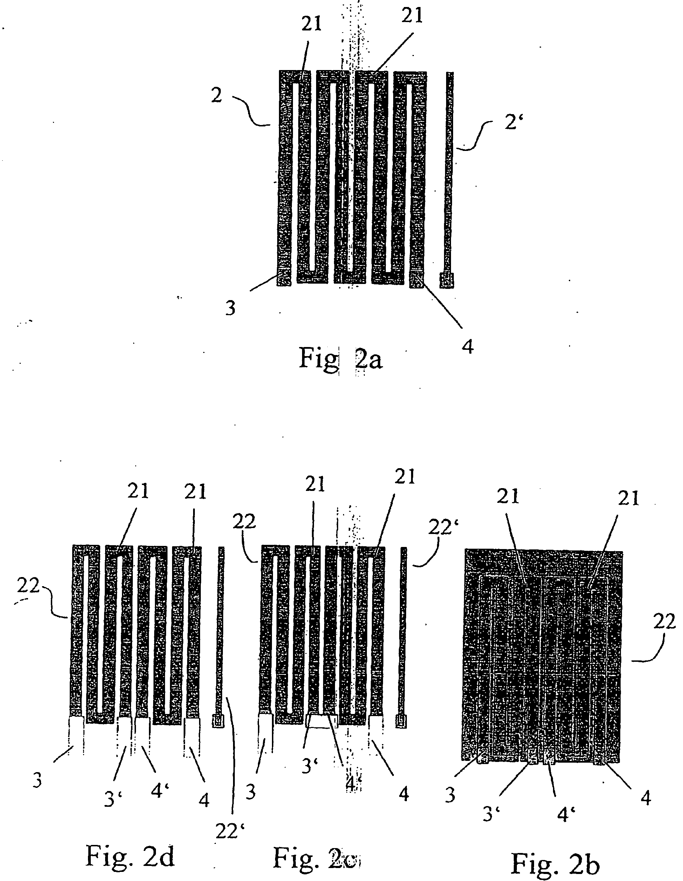

Fig. 2a zeigt ein Heizelement 2 in Form eines Mäanders mit sieben Bögen 21 und den beiden Kontakten 3, 4 in der Draufsicht und in der Seitenansicht 2'.

Fig. 2b zeigt ein Heizelement 22 in Form von zwei Mäandern mit je drei Bögen 21 und den elektrischen Kontakten 3,3',4'4.

Fig. 2c zeigt ein Heizelement 22 in Form von zwei Mäandern mit je drei Bögen 21 und den elektrischen Kontakten 3,3',4',4 in der Draufsicht und in der Seitenansicht 22'. Die Kontakte 3' und 4' sind elektrisch leitfähig miteinander verbunden. Wird zwischen den Kontakten 3 und 4 eine Spannung von 230 V angelegt und beträgt der Widerstand eines jeden Mäanders 20 kΩ, so ergibt sich nach P=U²/R=(230 V)²/(20 kΩ + 20 kΩ) etwa P=1,32 W an Heizleistung.

Fig. 2d zeigt ein Heizelement 22 in Form von zwei Mäandern mit je drei Bögen 21 und den elektrischen Kontakten 3,3',4',4 in der Draufsicht und in der Seitenansicht 22'. Wird zwischen den Kontakten 3 und 3' bzw. 4' und 4 je eine Spannung von 110 V angelegt und beträgt der Widerstand eines jeden Mäanders 20 kΩ, so ergibt sich nach P=U²/R=(110 V)²/(20 kΩ) + (110 V)²/(20 kΩ) etwa P=1,32 W an Heizleistung.

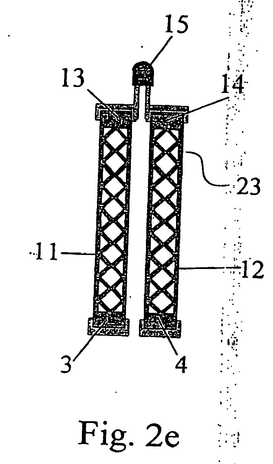

Fig. 2e zeigt ein Heizelement 23 in Form eines Gitters mit zwei Streifen 11,12. An den zwei korrespondierenden Enden der Streifen 11,12 befinden sich die Kontakte 3, 4. Die gegenüberliegenden Enden 13 und 14 sind über eine Leuchtdiode 15 elektrisch leitend verbunden.

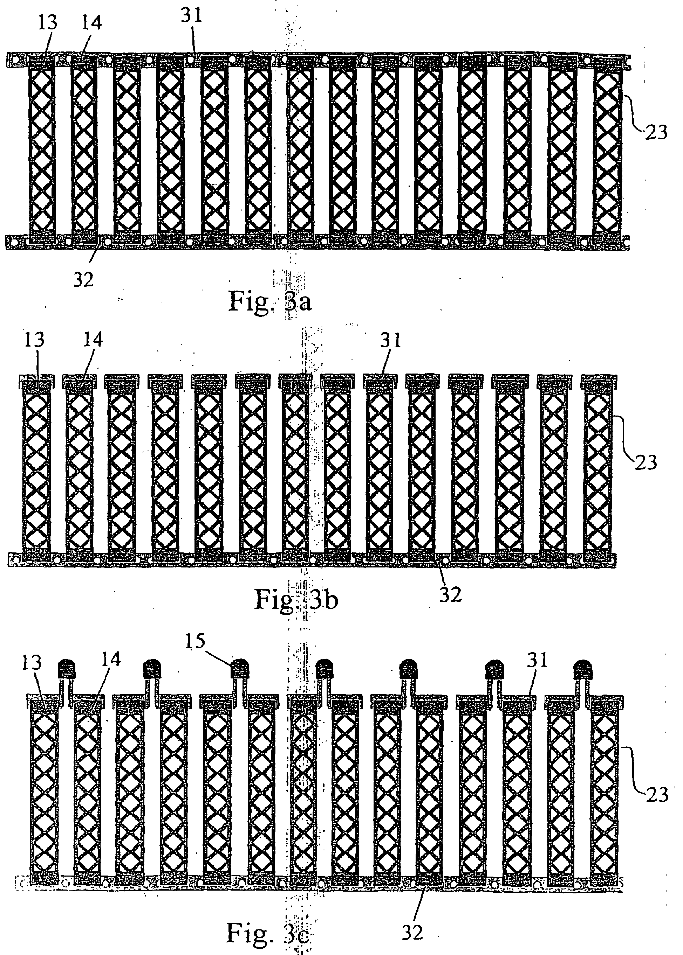

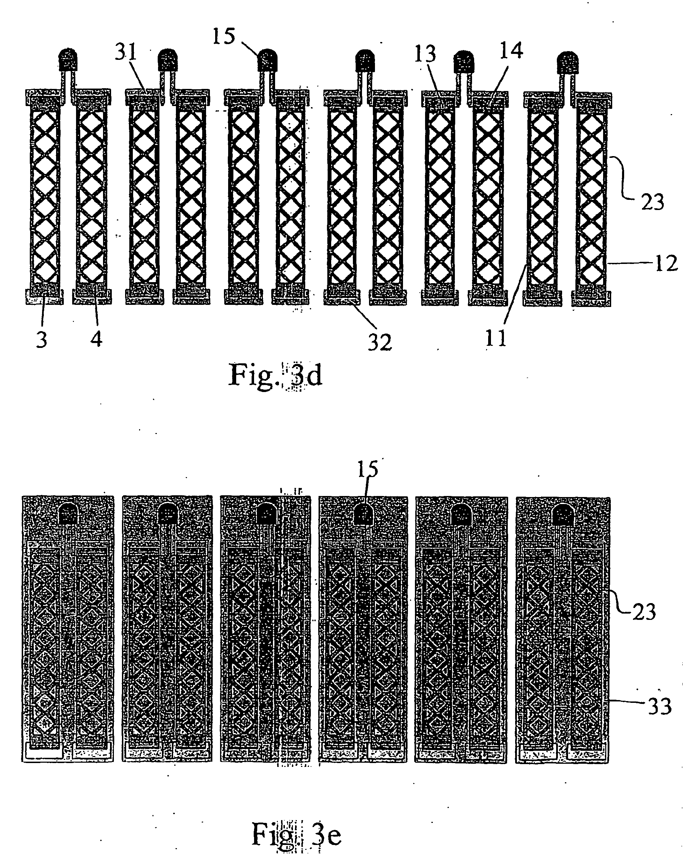

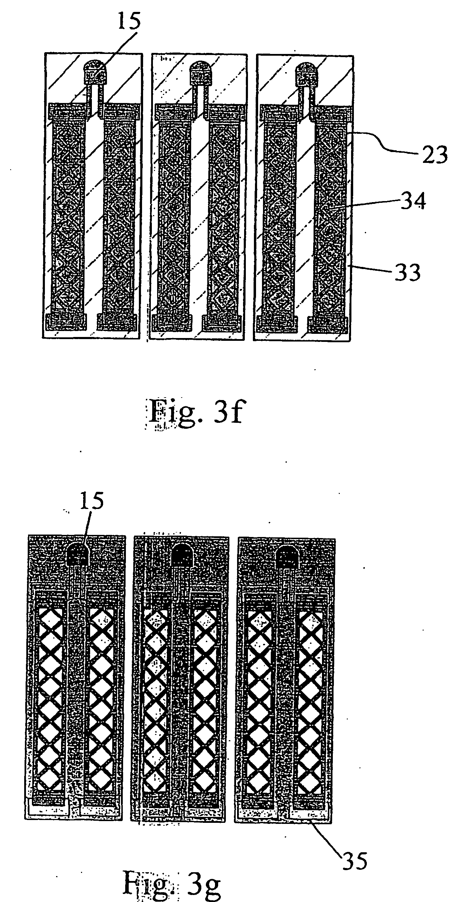

Die Schritte eines Verfahrens zur Herstellung eines Wirkstoffchips mit integriertem Heizelement und Leuchtdiode gemäß Fig. 2e sind in den Fig. 3a bis 3g dargestellt. ;

Zwei gelochte Messingbänder 31, 32 laufen durch eine Spritzmaschine und streifenförmige Heizelemente 23 aus leitfähigem Kunststoff in Form eines Gitters werden mit ihren Enden 13,14 an die Messingbänder angespritzt (Fig. 3a). Das Messingband 31 wird zwischen den Enden 13,14 durchtrennt (Fig. 3b). Zwischen die beiden freien Enden 13,14 wird eine LED 15 gelötet (Fig. 3c). Dann wird das Messingband 32 zwischen den Kontakten 3,4 getrennt und man erhält getrennte Heizelemente 23 (Fig. 3d). Anschließend wird jedes Heizelement 23 in ein Gehäuseunterteil 33 eingelegt (Fig. 3e) und der Wirkstoff 34 wird in das Gehäuse eingebracht (Fig. 3f). Zuletzt wird das Gehäuseoberteil 35 montiert (Fig. 3g).

### Beispiele

### Beispiel 1:

### Zusammensetzung und Anwendung eines erfindungsgemäßen Wirkstoffchips mit integriertem Heizgerät

In ein Kunststoff-Plättchen gemäß Fig. 1 aus Polypropylen-Material und von 70 mm Länge, 30 mm Breite und 5 mm Dicke wurde ein Heizelement 2 mit einem Querschnitt von 1 mm und einer Länge von 67 mm und einem elektrischen Widerstand von ca. 15 Ω eingegossen. Das Heizelement hatte die Form eines Mäanders. Das Kunststoffmaterial aus dem das Kunststoff-Plättchen bestand, enthielt zwischen 8,1 % und 8,4 %, insgesamt ca. 720 mg, des Wirkstoffs Transfluthrin.

Über die elektrischen Kontakte 3 und 4 wurde das Kunststoffplättchen über einen Adapter mit Netzgerät an eine Steckdose (230 V) angeschlossen. Die Spannung am elektrischen Widerstand des Wirkstoffchips betrug 230 V. Innerhalb von wenigen Minuten heizte sich der Draht in dem Kunststoffplättchen auf 65 bis 70°C auf und der Wirkststoff Transfluthrin begann in einer biologisch wirksamen Menge zu verdampfen. Über eine Zeitdauer von 8 Stunden wurde die Arbeitstemperatur im Bereich von 65 bis 90°C gehalten. Nach 45 dieser 8 Stunden-Zyklen konnte im Wirkstoffchip noch ein Anteil von ca. 70 % der ursprünglichen Wirkstoffmenge nachgewiesen werden. Zwischen zwei aufeinanderfolgenden Zyklen wurde eine Pause von 16 Stunden gemacht. Die Raumtemperatur betrug während des Versuchs 21 bis 25°C.

### Beispiel 2:

### Vergleich der Arbeistemperaturen von verschiedenen Verdampfersystemen

Die Eigenschaften eines erfindungsgemäße Wirkstoffchips mit integriertem Heizelement gemäß Beispiel 1 wurden mit den Eigenschaften von drei anderen Verdampfersystemen, die auch Langzeitsysteme sind, d.h. einen Wirkstoffvorrat für mehrere Tage haben, verglichen.

Als erstes Vergleichssystem wurde ein Gelverdampfer gewählt. 1, 6 g der Formulierung des Gelverdampfers hatten die folgende Zusammensetzung:

| | |
|---|---|
| 37,5 % reines Transfluthrin® = | 600,0 mg |
| 4,5 % Aerosil 200® = | 72,0 mg |
| 0,03 % Farbstoff Sudan Blau 670® = | 0,48 mg |
| 2,0 % Parfümöl Baygona 226863® = | 32,0 mg |
| 55,97 % Diphyl THT ® | 895,52mg |
| = | **1600,00 mg** |

Die Verdampfung erfolgte durch ein entsprechendes Heizgerät bei einer Temperatur von 100 bis 110°C.

Als zweites Vergleichssystem wurde ein Flüssigverdampfer gewählt. 35 g der Formulierung des Flüssigverdampfers hatten die folgende Zusammensetzung:

| | |
|---|---|
| 0,88 % Transfluthrin | 0,308 g |
| 67,12 % Isopar M | 23,492 |
| 30,0 % Isopar V | 10,5 g |
| 1,0 % Butylhydroxytoluol | 0,35 g |
| 1,0 % Parfümöl Deodorins B.Y.R. N 3 | 0,35 g |
| | **35,0** |

Die Verdampfung erfolgte durch ein entsprechendes Heizgerät bei einer Temperatur von 125 bis 135°C.

Als drittes Vergleichssystem wurde ein polymerer Wirkstoffträger mit externem Heizgerät gewählt. Es wurden dasselbe Kunststoffmaterial und dieselbe Wirkstoffmenge wie in Beispiel 1 verwendet.

Die Verdampfung erfolgte durch ein entsprechendes Heizgerät bei Temperaturen von 100°C und 150°C.

Alle Versuche wurden bei einer Raumtemperatur von 21 bis 25°C durchgeführt.

In Tab. 1 sind die für die verschiedenen Verdampfersysteme gemessenen Arbeitstemperaturen aufgeführt. Die Arbeitstemperatur ist diejenige Temperatur, bei der eine ausreichende biologische Wirkung auftritt. Der in Tab. 1 dargestellte Vergleich zeigt, dass die Arbeitstemperatur des erfindungsgemäßen Wirkstoffchips mit integriertem Heizelement mit 65 bis 90°C deutlich unter der Arbeitstemperatur der bekannten Verdampfersysteme liegt. Der polymere Wirkstoffträger, der dieselbe Zusammensetzung hat wie der erfindungsgemäße Wirkstoffchip gemäß Beispiel 1 und nur abweichend zu dem erfindungsgemäßen Wirkstoffchip kein integriertes Heizgerät hat, sondern ein externes Heizgerät, zeigte eine Arbeitstemperatur im Bereich von 140°C bis 150°C. Bei Temperaturen im Bereich von 110°C und 100°C nahm die biologische Wirkung merklich ab. Der Plättchenverdampfer wurde in seiner kommerziell erhältlichen Form (PV 3 Heizer, Fa. DBK) verwendet.

**Tab. 1: Arbeitstemperatur verschiedener Verdampfersysteme im Vergleich**

| System | Temperfurbereich |
|---|---|
| Plättchenverdampfer | 140-150°C |
| Flüssigverdampfer | 125-135°C |
| Gelverdampfer | 100-110°C |
| erfindungsgemäßer Wirkstoffchip | 65-90°C |
| Polymerer Wirkstoffträger | 140-150°C |

### Beispiel 3:

### Vergleich der Abdampfraten von verschiedenen Verdampfersystemen

Es wurde ein Langzeittest der Abdampfrate von Wirkstoff im Vergleich zwischen dem erfindungsgemäßen Wirkstoffchip und einem Gelverdampfer und einem Flüssigverdampfer durchgeführt.

Die Zyklendauer betrug 8 Stunden mit 16 Stunden Unterbrechung zwischen zwei aufeinanderfolgenden Zyklen.

Die Arbeitstemperatur der Systeme wurde gemäß Tab. 1 so gewählt, dass eine vergleichbare biologische Wirkung erzielt werden konnte.

Die Ergebnisse des Vergleichs der Äbdampfraten über 45 Zyklen sind in den Tabellen 2 bis 5 dargestellt. Tab. 2 gibt die Abgaberaten der Gesamtformulierungen und Tab. 3 die Mittelwerte dieser Abgaberaten wieder. Tab. 4 gibt an, wieviel Wirkstoff in den einzelnen Zyklen abgegeben wurde, und Tab. 5 zeigt den Mittelwert der Abgaberaten des Wirkstoffs.

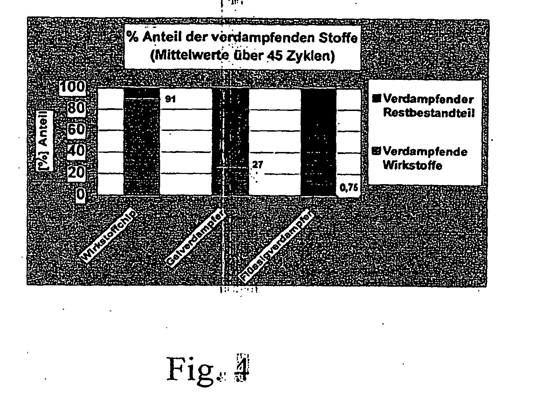

Der Gewichtsverlust an der Gesamtformulierung der einzelnen Systeme setzt sich zusammen aus abgedampftem Wirkstoff und Abdampfung von zusätzlichen Bestandteilen der Formulierung. Die abgedampfte Menge an der Gesamtformulierung liegt beim Gelverdampfer und beim Flüssigverdampfer deutlich höher als beim erfindungsgemäßen Wirkstoffchip (Tab. 2). Ein Vergleich der Menge an abgedampftem Wirkstoff zeigt, dass die abgedampfte Wirkstoffmenge beim erfindungsgemäßen Wirkstoffchip der abgedampften Wirkstoffmenge beim Gelverdampfer und beim Flüssigverdampfer bis auf 1 bis 2 mg/Zyklus entspricht (Tab. 4). Dies bestätigt, dass die gewählten Arbeitstemperaturen zu vergleichbaren biologischen Wirkungen, bedingt durch eine vergleichbare abgedampfte Wirkstoffmenge, führen. Betrachtet man jedoch den Anteil von Wirkstoff an der gesamten abgedampften Menge, so zeigt sich, dass dieser Anteil beim erfindungsgemäßen Wirkstoffchip ab dem 4. Zyklus bei 100 % liegt, für den Gelverdampfer zwischen 25 und 35 % und für den Flüssigverdampfer bei unter 1 %. Aus Tab. 6 geht hervor, wieviel Wirkstoff im Verhältnis zur insgesamt abgedampften Menge verdampft wurde. Dies waren beim erfindungsgemäßen Wirkstoffchip über alle 45 Zyklen im Mittel 91 %, beim Gelverdampfer 27 % und bein Flüssigverdampfer 0,75 %. In den Anfangszyklen (1. bis 7. Zyklus) ist der Wirkstoffanteil bei allen drei Systemen geringer als in den letzten Zyklen (40. bis 45. Zyklus). In Fig. 4 wird veranschaulicht, wie sich die gesamte abgedampfte Stoffmenge für die drei getesteten Systeme aus Wirkstoffanteiten und übrigen Anteilen zusammensetzt.

Das gute Verhältnis von Wirkstoff zur gesamten abgedampften Menge beim erfindungsgemäßen Wirkstoffchip ist auf die Formulierung, verbunden mit der niedrigen Arbeitstemperatur, die durch das integrierte Heizgerät möglich wird, zurückzuführen. Die Verdampfungstemperatur für das Polypropylen-Material, in das der Wirkstoff eingebettet ist, liegt deutlich über 100°C, während für die Verdampfung einer ausreichenden Menge an Wirkstoff eine Arbeitstemperatur von unter 100°C ausreicht. In allen Vergleichssystemen liegt die Temperatur, die für die Verdampfung einer ausreichenden Menge an Wirkstoff notwendig ist, auch bei über 100°C, so dass automatisch mit dem Wirkstoff große Anteile des übrigen Materials verdampft werden.

Der fast 100%ige Wirkstoffanteil an der gesamten abgedampften Menge beim erfindungsgemäßen Wirkstoffchip hat den Vorteil der geringeren Belastung der Umwelt bei vergleichbarer biologischer Wirksamkeit im Vergleich zu den bekannten Verdampfersystemen. Die im Vergleich geringere Belastung der Umwelt mit dem erfindungsgemäßen Wirkstoffchip manifestiert sich auch in der gleichmäßigen, niedrigen Abdampfrate.

Die Abdampfrate über die gesamte Versuchszeit für den erfindungsgemäßen Wirkstoffchip hat eine absolute Schwankungsbreite, die deutlich unter der Schwankungsbreite für den Gelverdampfer und den Flüssigverdampfer liegt (Tab. 3). Die absolute Schwankungsbreite der Abdampfrate an Wirkstoff über die gesamte Versuchszeit ist in allen drei untersuchten Fällen vergleichbar und liegt zwischen 0,6 mg/Zyklus (Flüssigverdampfer)und 0,9 mg/Zyklus (Gelverdampfer) (Tab. 4).

**Tab. 2: Abgaberate der Gesamtformulierung**

| Zyklus | erfindungsgemäßer Wirkstoffchip Gewichtsverlust [mg/Zyklus] | Gelverdampfer Gewichtsverlust [mg/Zyklus] | Flüssigverdampfer Gewichtsverlust [mg/Zyklus] |
|---|---|---|---|
| 1 | 17,6 | 37 | 1010 |
| 2 | 11,2 | 38 | 890 |
| 3 | 11,3 | 33 | 887 |
| 4 | 7,5 | 33 | 885 |
| 5 | 6,8 | 26 | 880 |
| 6 | 5,4 | 26 | 870 |
| 7 | 2,8 | 26 | 860 |
| 8 | 6,7 | 26 | 850 |
| 9 | 4,3 | 26 | 850 |
| 10 | 5,7 | 26 | 840 |
| 11 | 5,7 | 26 | 840 |
| 12 | 5,3 | 22 | 840 |
| 13 | 4,8 | 22 | 830 |
| 14 | 6,2 | 22 | 820 |
| 15 | 5,0 | 22 | 820 |
| 16 | 3,6 | 22 | 820 |
| 17 | 2,6 | 22 | 820 |
| 18 | 3,0 | 19 | 820 |
| 19 | 4,3 | 19 | 820 |
| 20 | 4,1 | 18 | 800 |
| 21 | 3,8 | 18 | 800 |
| 22 | 4,2 | 16 | 800 |
| 23 | 3,5 | 16 | 790 |
| 24 | 3,2 | 16 | 790 |
| 25 | 3,2 | 16 | 790 |
| 26 | 3,1 | 16 | 780 |
| 27 | 3,0 | 16 | 770 |
| 28 | 3,5 | 17 | 770 |
| 29 | 4,5 | 17 | 770 |
| 30 | 4,3 | 11 | 770 |
| 31 | 3,6 | 11 | 770 |
| 32 | 3,4 | 10 | 770 |
| 33 | 2,4 | 10 | 770 |
| 34 | 3,4 | 10 | 770 |
| 35 | 4,3 | 10 | 780 |
| 36 | 1,9 | 10 | 760 |
| 37 | 1,8 | 10 | 760 |
| 38 | 1,8 | 10 | 760 |
| 39 | 2,5 | 10 | 770 |
| 40 | 2,7 | 9 | 775 |
| 41 | 2,8 | 9 | 760 |
| 42 | 3,0 | 7 | 760 |
| 45 | 3,0 | 6 | 680 |

**Tab. 3: Mittelwerte der Abgaberate der Gesamtformulierung**

| System | Mittelwert [mg]/Zyklus | Sitandardabweichung ± [mg]/Zyklus |
|---|---|---|
| erfindungsgemäßer Wirkstoffchip | 4,6 | 1,45 |
| Gelverdampfer | 18,4 | 4,1 |
| Flüssigverdampfer | 809 | 28 |

**Tab. 4: Abgaberate des Wirkstoffs**

| Zyklus | erfindungsgemäßer Wirkstoffchip Gewichtsverlust [mg/Zyklus] | Gelverdampfer Gewichtsverlust [mg/Zyklus] | Flüssigverdampfer Gewichtsverlust [mg/Zyklus] |
|---|---|---|---|
| 1 | 7,1 | 9,3 | 6,8 |
| 2 | 7,2 | 9,5 | 7 |
| 3 | 7,3 | 7,6 | 7,5 |
| 4 | 7,5 | 7,6 | 6,5 |
| 5 | 6,8 | 6,8 | 7,9 |
| 6 | 5,4 | 6,6 | 7,2 |
| 7 | 2,8 | 6,7 | 5,5 |
| 8 | 6,7 | 6,7 | 7,6 |
| 9 | 4,3 | 6,8 | 6,5 |
| 10 | 5,7 | 6,9 | 7,6 |
| 11 | 5,7 | 7,1 | 7,5 |
| 12 | 5,3 | 5,9 | 5,3 |
| 13 | 4,8 | 5,5 | 7,4 |
| 14 | 6,2 | 5,7 | 4,8 |
| 15 | 5 | 5,8 | 6,5 |
| 16 | 3,6 | 5,9 | 6,4 |
| 17 | 2,6 | 6 | 6,3 |
| 18 | 3 | 4,7 | 7,1 |
| 19 | 4,3 | 4,5 | 6,5 |
| 20 | 4,1 | 4,4 | 7,2 |
| 21 | 3,8 | 4,3 | 7 |
| 22 | 4,2 | 4,1 | 7 |
| 23 | 3,5 | 4,2 | 7,1 |
| 24 | 3,2 | 4,3 | 7,1 |
| 25 | 3,2 | 4,4 | 7 |
| 26 | 3,1 | 4,1 | 7,1 |
| 27 | 3 | 4,2 | 6,9 |
| 28 | 3,5 | 4,4 | 6,2 |
| 29 | 4,5 | 4,3 | 6,1 |
| 30 | 4,3 | 3,9 | 5,8 |
| 31 | 3,6 | 3,8 | 5,6 |
| 32 | 3,4 | 3,3 | 5,2 |
| 33 | 2,4 | 3,2 | 5,1 |
| 34 | 3,4 | 3,2 | 5 |
| 35 | 4,3 | 3,1 | 4,5 |
| 36 | 1,9 | 3 | 4,4 |
| 37 | 1,8 | 3,3 | 4,3 |
| 38 | 1,8 | 3,1 | 4,2 |
| 39 | 2,5 | 3 | 4,1 |
| 40 | 2,7 | 2,7 | 4 |
| 41 | 2,8 | 2,5 | 3,9 |
| 42 | 3 | 2,4 | 3,8 |
| 45 | 3 | 2,3 | 3,8 |

**Tab. 5: Mittelwerte der Abgaberate des Wirkstoffs**

| | Mittelwert [mg]/Zyklus | Standardabweichung +/- [mg]/Zyklus |
|---|---|---|
| erfindungsgemäßer Wirkstoffchip | 4,2 | 0,8 |
| Gelverdampfer | 4,9 | 4,9 |
| Flüssigverdampfer | 6,1 | 0,6 |

**Tab. 6: Anteil des Wirkstoffes an der Gesamtmenge der verdampfenden Stoffe (Mittelwert über 45 Zyklen) in (%)**

| | ∅ über 45 Zyklen | Anfangszyklen (1-7) | Endzyklen (40-45) |
|---|---|---|---|
| erfindungsgemäßer Wirkstoffchip | 91 | 86 | 100 |
| Gelverdampfer | 27 | 25 | 70 |
| Flüssigverdampfer | 0,75 | 0,5 | 3,0 |

### Beispiel 4:

### Vergleich der für eine vergleichbare biologische Wirkung notwendigen Wirkstoffmengen von verschiedenen Verdampfersystemen

Ein Vorteil des erfindungsgemäßen Wirkstoffchips zeigt sich darin, dass eine gleiche abgedampfte Wirkstoffmenge wie beim Gelverdampfer und beim Flüssigverdampfer eine bessere biologische Wirkung hat. Dies liegt daran, dass beim Gelverdampfer und beim Flüssigverdampfer ein Teil des abgedampften Wirkstoffs durch Kondensation an kühlen Stellen des Heizgeräts direkt wieder verloren geht, während der abgedampfte Wirkstoff beim erfindungsgemäßen Wirkstoffchip fast vollständig ausgenutzt wird. Tab. 7 gibt an, wieviel Wirkstoff mit dem erfindungsgemäßen Wirkstöffchip, dem Gelverdampfer und dem Flüssigverdampfer jeweils verdampft werden muss, damit eine vergleichbare biologische Wirkung eintritt. Eine Reduktion der benötigten abgedampften Wirkstoffmenge wirkt sich positiv auf die Verringerung der Umweltbelastung, die Langlebigkeit und die Temperafür aus.

**Tab. 7: Wirkstoffmenge, die eine gleiche biologische Wirkung ergibt**

| Menge pro Zyklus | | |
|---|---|---|
| [mg/8h] | | |
| erfindungsgemäßer Wirkstoffchip | Gelverdampfer | Flüssigverdampfer |
| 4,2 | 4,9 | 6,1 |

| Menge pro Stunde (Index) | | |
|---|---|---|
| [mg/h1] | | |
| erfindungsgemäßer Wirkstoffchip | Gelverdampfer | Ftüssigverdampfer |
| 0,5 (100) | 0,6 (120) | 0,8 (160) |

### Beispiel 5:

### Biologische Wirkung der Wirkstoffchips

Die biologische Wirkung von erfindungsgemäßen Wirkstoffchips mit integriertem Heizelement auf Mücken der Art Aedes Aegypti sensibel wurde in Beispiel 5 nachgewiesen.

Der Versuch wurde in einem Raum von 36 m³ Größe, mit einem offenstehenden Fenster bei einer Temperatur von 20 bis 28°C und einer Rel: Raumfeuchte von 17 bis 34 % durchgeführt. Die Arbeitstemperatur betrug 65 bis 90°C. Es wurden erfindungsgemäße Wirkstoffchips gemäß Beispiel 1 verwendet.

**Tab. 8: Biologische Wirkung des erfindungsgemäßen Wirkstoffchips**

| Betriebsdauer/ Prüfung nach Tagen (Stunden) | Mückenansatz nach Stunden | Formulierung Beispiel 1 Knockdown Wirkung n. Min. bzw. % tot nach h | | | |
|---|---|---|---|---|---|
| | | 50% | 100% | 9h | 24h |
| 1. Tag | 0 | 46' | 55' | 100 | 100 |
| | 1 | 16' | 32 | 100 | 100 |
| | 2 | 9' | 28' | 100 | 100 |
| | 3 | 11' | 23' | 100 | 100 |
| | 4 | 13' | 19' | 100 | 100 |
| | 5 | 12' | 23' | 100 | 100 |
| 8 Stunden | 6 | 23' | 43' | 100 | 100 |
| | 7 | 33' | 1h00' | 100 | 100 |
| | 8 | 26' | >8h | 88 | 100 |
| 2 Tage | 0 | 44' | 1h14' | 100 | 100 |
| | 1 | 59' | 1h19' | 100 | 100 |
| | 2 | 36' | 1h03' | 100 | 100 |
| | 3 | 19' | 49' | 100 | 100 |
| | 4 | 19' | 53' | 100 | 100 |
| | 5 | 46' | 1h12' | 100 | 100 |
| | 6 | 29' | 1h17' | 00 | 100 |
| | 7 | 24' | 43' | 100 | 100 |
| 16 Stunden | 8 | 14' | 32' | 100 | 100 |
| 3 Tage | 0 | 40' | 48' | 100 | 100 |
| | 1 | 9' | 13' | 100 | 100 |
| | 2 | 3' | 5' | 100 | 100 |
| | 3 | 3' | 6' | 100 | 100 |
| | 4 | 2' | 4' | 100 | 100 |
| | 5 | 3' | 7' | 100 | 100 |
| | 6 | 5' | 11' | 100 | 100 |
| | 7 | 7' | 16' | 100 | 100 |
| 24 Stunden | 8 | 4' | >1h | 90 | 100 |

Die Ergebnisse zeigen die erwartete biologische Wirkung des Systems. Die Anwendungszeit kann durch Variation der Wirkstoffkonzentration im erfindungsgemäßen Wirkstoffchip beliebig eingestellt werden.

## Patentansprüche

1. Wirkstoffchip (1) mit mindestens einem integrierten Heizelement (2), bestehend aus einem leitfähigen, maschinell verarbeitbaren Material und zwei elektrischen Kontakten (3, 4), aus dem der Wirkstoff durch Aufheizen des Chips (1) durch Anlegen einer elektrischen Spannung an die elektrischen Kontakte (3, 4) verdampft werden kann,
**dadurch gekennzeichnet, dass** das Heizelement (2) in den Wirkstoff oder ein Wirkstoffgemisch, gegebenenfalls in Kombination mit einem Wirkstoffträger, so eingebettet ist, dass das Ganze eine Einheit bildet.

2. Wirkstoffchip nach Anspruch 1, **dadurch gekennzeichnet, dass** er eine rechteckige Platte bildet und eine Lange im Bereich von 10 bis 100 mm, eine Breite im Bereich von 5 bis 100 mm und eine Dicke im Bereich von 3 bis 20 mm aufweist.

3. Wirkstoffchip nach Anspruch 1 oder 2, **dadurch gekennzeichnet, dass** das Heizelement seiner Länge nach von dem den Wirkstoff enthaltenden Chipteil umschlossen ist, so dass sich die Form des Heizelements auf der äu-ßeren Form des Wirkstoffchips abbildet.

4. Wirkstoffchip nach einem der Ansprüche 1 bis 3, **dadurch gekennzeichnet, dass** der Wirkstoff oder das Wirkstoffgemisch in flüssiger oder in fester Form vorliegt und mit einer für den Wirkstoff oder das Wirkstoffgemisch in flüssiger und fester Form undurchdringbaren und in gasförmiger Form durchdringbaren Oberflächenschicht versehen ist.

5. Wirkstoffchip nach einem der Ansprüche 1 bis 4, **dadurch gekennzeichnet, dass** der Wirkstoff oder das Wirkstoffgemisch flüssig, gelförmig oder fest ist und in einem Gehäuse vorliegt und das Heizelement in ihn eingebettet ist.

6. Wirkstoffchip nach einem der Ansprüche 1 bis 5, **dadurch gekennzeichnet, dass** der Wirkstoff oder das Wirkstoffgemisch an einen Wirkstoffträger gebunden ist.

7. Wirkstoffchip nach Anspruch 6, **dadurch gekennzeichnet, dass** der Wirkstoffträger ein Polymer ist.

8. Wirkstoffchip nach Anspruch 6 oder 7, **dadurch gekennzeichnet, dass** der Chip im wesentlichen aus dem Heizelement und dem diesen umgebenden Wirkstoffträger mit Wirkstoff oder Wirkstoffgemisch besteht.

9. Wirkstoffchip nach einem der Ansprüche 6 bis 8, **dadurch gekennzeichnet, dass** der Wirkstoffträger aus Kunststoffmaterialien wie Polyethylen, Polypropylen, TPX®-Typen, Desmopan® 8410, Vestamid® 1800, BAK® 402-005 besteht und dass der Wirkstoff aus Transfluthrin® besteht.

10. Wirkstoffchip nach einem der Ansprüche 1 bis 9, **dadurch gekennzeichnet, dass** das Heizelement aus Keramik, einem Heizleiter, einer bedampften Folie oder einem leitfähigen Kunststoff besteht.

11. Wirkstoffchip nach Anspruch 10, **dadurch gekennzeichnet, dass** das Heizelement aus einem Heizwiderstand oder PTC besteht.

12. Wirkstoffchip nach einem der Ansprüche 1 bis 11, **dadurch gekennzeichnet, dass** das Heizelement einen Widerstand von 10 kΩ bis 100 kΩ aufweist.

13. Wirkstoffchip nach einem der Ansprüche 1 bis 11, **dadurch gekennzeichnet, dass** das Heizelement einen Widerstand von 2 kΩ bis 30 kΩ aufweist.

14. Wirkstoffchip nach einem der Ansprüche 1 bis 13, **dadurch gekennzeichnet, dass** die Heizleistung des Heizelements zwischen 0,1 W und 5 W liegt.

15. Wirkstoffchip nach einem der Ansprüche 1 bis 14, **dadurch gekennzeichnet, dass** mit dem Heizelement eine Temperatur im Bereich von 60 °C bis 140 °C eingestellt werden kann.

16. Wirkstoffchip nach einem der Ansprüche 1 bis 15, **dadurch gekennzeichnet, dass** das Heizelement (2) in Form eines Mäanders mit mindestens einem Bogen (21) angeordnet ist, wobei sich die beiden elektrischen Kontakte (3, 4) an den beiden Enden des Mäanders befinden.

17. Wirkstoffchip nach einem der Ansprüche 1 bis 15, **dadurch gekennzeichnet, dass** das Heizelement (22) in Form von zwei Mäandern mit mindestens einem Bogen (21) angeordnet ist, wobei sich jeweils ein elektrischer Kontakt (3,3'; 4,4') an jedem Ende der beiden Mäander befindet.

18. Wirkstoffchip nach Anspruch 17, **dadurch gekennzeichnet, dass** die einander zugewandten elektrischen Kontakte (3',4') der beiden Mäander elektrisch leitend miteinander verbunden sind.

19. Wirkstoffchip nach einem der Ansprüche 1 bis 18, **dadurch gekennzeichnet, dass** das Heizelement (23) eine Gitter- oder Wabenstruktur hat.

20. Wirkstoffchip nach Anspruch 19, **dadurch gekennzeichnet, dass** das Heizelement (23) mit der Gitter- oder Wabenstruktur in zwei Streifen (11, 12) ausgeführt ist, die jeweils an einem Ende kontaktiert und an dem der Kontaktierung entgegengesetzten Ende elektrisch leitend miteinander verbunden sind.

21. Wirkstoffchip nach einem der Ansprüche 1 bis 20, **dadurch gekennzeichnet, dass** die elektrischen Kontakte aus Messingblech oder Kupfer bestehen.

22. Wirkstoffchip nach einem der Ansprüche 1 bis 21, **dadurch gekennzeichnet, dass** eine Betriebsanzeige (15) in den Chip integriert ist.

23. Wirkstoffchip nach Anspruch 22, **dadurch gekennzeichnet, dass** die Betriebsanzeige (15) eine bipolare LED ist.

24. Wirkstoffchip nach einem der Ansprüche 1 bis 23, **dadurch gekennzeichnet, dass** zwischen die Stromversorgung und den Kontakt ein an sich bekannter Timer-Chip eingesetzt und in den Wirkstoffchip ein zusätzlicher Widerstand integriert ist.

25. Wirkstoffchip nach einem der Ansprüche 1 bis 24, **dadurch gekennzeichnet, dass** er als Wirkstoffe Pyrethroide, acaride Wirkstoffe, Duftstoffe oder ätherische Öle enthält.

26. Wirkstoffchip nach Anspruch 25, **dadurch gekennzeichnet, dass** er als Wirkstoff Transfluthrin® oder Pynamin forte® oder Benzylbenzoat enthält.

27. Wirkstoffchip nach einem der Ansprüche 1 bis 26, **dadurch gekennzeichnet, dass** der Wirkstoff oder das Wirkstoffgemisch bei einer Temperatur von unter 100 °C, bevorzugt im Bereich von 65 °C bis 90 °C, verdampft werden kann.

28. Wirkstoffchip nach einem der Ansprüche 1 bis 27, **dadurch gekennzeichnet, dass** nach einer Anfangsphase von 5 Zyklen ä 8 Stunden die abgedampfte Menge an Gesamtformulierung im Mittel über einen Zyklus mindestens 70 %, bevorzugt mehr als 90 besonders bevorzugt mehr als 99 % Wirkstoff oder Wirkstoffgemisch enthält.

29. Verfahren zur Herstellung eines Wirkstoffchips nach den Ansprüchen 1 bis 28, **dadurch gekennzeichnet, dass** mindestens ein Heizelement (23) an ein Endlosmetallband angespritzt wird die einzelnen Heizelemente (23) aus dem Endlosmetallband ausgestanzt, die Kontakte (13, 14) getrennt und die Heizelemente (23) in ein Gehäuse eingelegt werden, der Wirkstoff oder das Wirkstoffgemisch (34) zu dem mindestens einen Heizelement (23) in das Gehäuse (33, 35) gegeben und das Gehäuse geschlossen wird.

30. Verfahren zur Herstellung eines Wirkstoffchips nach den Ansprüchen 1 bis 28, **dadurch gekennzeichnet, dass** mindestens ein Heizelement (23) an ein Endlosmetallband angespritzt wird, die einzelnen Heizelemente (23) aus dem Endlosmetallband ausgestanzt, die Kontakte (13,14) getrennt und in ein den Wirkstoff oder das Wirkstoffgemisch (34) enthaltendes Gehäuse (33, 35) eingelegt werden und das Gehäuse geschlossen wird.

31. Verfahren zur Herstellung eines Wirkstoffchips mit einem Heizelement (23), bestehend aus zwei Streifen (13, 14) nach Anspruch 20, **dadurch gekennzeichnet, dass** jeder Streifen (13, 14) an zwei ndlosmetallbänder (31, 32) angespritzt wird, das eine Endlosmetallband (31) zwischen je zwei Streifen durchtrennt wird, das andere Endlöshietallband (32) zwischen jedem Streifen durchtrennt wird und die auf diese Weise hergestellten Heizelemente (23) in ein Gehäuse (33, 35), das den Wirkstoff oder das Wirkstoffgemisch (34) enthält, eingelegt werden und das Gehäuse geschlossen wird.

32. Verfahren zur Herstellung eines Wirkstoffchips mit einem Heizelement (23), bestehend aus zwei Streifen (13,14) nach Anspruch 31, **dadurch gekennzeichnet, dass** das erste Metallband (31) zwischen jedem Streifen durchtrennt wird und zwischen je zwei Streifen eine LED (15) eingelötet wird.

33. Verfahren zur Herstellung eines Wirkstoffchips, bei dem der Wirkstoff an einen Wirkstoffträger gebunden ist, nach den Ansprüchen 6 bis 9, **dadurch gekennzeichnet, dass** ein Heizetemeht (23) an mindestens ein Endlosmetaltband angespritzt wird, der Wirkstoffträger um das Heizelement am Endlosmetallband gespritzt wird und dann die einzelnen Wirkstoffchips aus dem Band von Wirkstoffträgern am Endtosmetattbänd ausgestanzt und die Kontakte getrennt werden.

34. Verfahren zur Herstellung eines Wirkstoffchips, bei dem der Wirkstoff an einen Wirkstoffträger gebunden ist, nach den Ansprüchen 6 bis 9, **dadurch gekennzeichnet, dass** der Wirkstoffträger ein Polymer und das Heizelement ein leitfähiger Kunststoff ist und das Heizelement zusammen mit dem Wirkstoffträger extrudiert wird, so dass ein Kunststoffdraht erhalten wird, dessen Kern das Heizelement bildet und dessen Mantel der Wirkstoffträger bildet.

## Claims

1. An active compound chip (1) comprising at least one integrated heating element (2) consisting of a conductive, mechanically processable material and two electrical contacts (3, 4), wherefrom the active compound can be evaporated by heating chip (1) by applying an electrical voltage to the electrical contacts (3, 4),
**characterized in that** the heating element (2) is embedded into the active compound or a mixture of active compounds, optionally in combination with a carrier for the active compound(s), in such manner, that an integral unit is obtained.

2. The active compound chip as claimed in claim 1, **characterized in that** it forms a rectangular plate and has a length in the range of from 10 to 100 mm, a breadth in the range of from 5 to 100 mm and a thickness in the range of from 3 to 20 mm.

3. The active compound chip as claimed in claim 1 or 2, **characterized in that** the heating element is surrounded, according to its length, by the chip part comprising the active compound so that the shape of the heating element forms an image on the external shape of the active compound chip.

4. The active compound chip as claimed in one of claims 1 to 3, **characterized in that** the active compound or the mixture of active compounds is present in liquid or in solid form and is provided with a surface layer which is impenetrable to the active compound or the mixture of active compounds in liquid and solid form and penetrable in gaseous form.

5. The active compound chip as claimed in one of claims 1 to 4, **characterized in that** the active compound or the mixture of active compounds is liquid, gelatinous or solid and is present in a housing and the heating element is embedded in it.

6. The active compound chip as claimed in one of claims 1 to 5, **characterized in that** the active compound or the mixture of active compounds is bound to an active compound carrier.

7. The active compound chip as claimed in claim 6, **characterized in that** the active compound carrier is a polymer.

8. The active compound chip as claimed in claim 6 or 7, **characterized in that** the chip essentially consists of the heating element and the active compound carrier containing the active compound or the mixture of active compounds surrounding the heating element.

9. The active compound chip as claimed in one of claims 6 to 8, **characterized in that** the active compound carrier consists of plastic materials such as polyethylene, polypropylene, ®TPX types, ®Desmopan 8410, ®Vestamid 1800, ®BAK 402-005 and **in that** the active compound consists of ®Transfluthrin.

10. The active compound chip as claimed in one of claims 1 to 9, **characterized in that** the heating element consists of ceramic, a heat conductor, a vapor-deposited film or a conductive plastic.

11. The active compound chip as claimed in claim 10, **characterized in that** the heating element consists of a heating resistance or PTC.

12. The active compound chip as claimed in one of claims 1 to 11, **characterized in that** the heating element has a resistance of from 10 kΩ to 100 kΩ.

13. The active compound chip as claimed in one of claims 1 to 11, **characterized in that** the heating element has a resistance of from 2 kΩ to 30 kΩ.

14. The active compound chip as claimed in one of claims 1 to 13, **characterized in that** the heating power of the heating element is between 0.1 W and 5 W.

15. The active compound chip as claimed in one of claims 1 to 14, **characterized in that** a temperature in the range of from 60 °C to 140 °C can be established using the heating element.

16. The active compound chip as claimed in one of claims 1 to 15, **characterized in that** the heating element (2) is arranged in the form of a meander having at least one bend (21), the two electrical contacts (3, 4) being located on the two ends of the meander.

17. The active compound chip as claimed in one of claims 1 to 15, **characterized in that** the heating element (22) is arranged in the form of two meanders having at least one bend (21), one electrical contact (3,3', 4,4') in each case being located on each end of the two meanders.

18. The active compound chip as claimed in claim 17, **characterized in that** the electrical contacts (3',4') facing one another of the two meanders are connected to one another in an electrically conducting manner.

19. The active compound chip as claimed in one of claims 1 to 18, **characterized in that** the heating element (23) has a lattice or honeycomb structure.

20. The active compound chip as claimed in claim 19, **characterized in that** the heating element (23) having the lattice or honeycomb structure is designed in two strips (11, 12) which in each case contact at one end and are connected to one another in an electrically conducting manner at the end opposite to the contacting.

21. The active compound chip as claimed in one of claims 1 to 20, **characterized in that** the electrical contacts consist of sheet brass or copper.

22. The active compound chip as claimed in one of claims 1 to 21, **characterized in that** an operating display (15) is integrated into the chip.

23. The active compound chip as claimed in claim 22, **characterized in that** the operating display (15) is a bipolar LED.

24. The active compound chip as claimed in one of claims 1 to 23, **characterized in that** a timer chip known per se is employed between the current supply and the contact and an additional resistance is integrated into the active compound chip.

25. The active compound chip as claimed in one of claims 1 to 24, **characterized in that** it contains pyrethroids, acaricidal active compounds, fragrances or ethereal oils as the active compounds.

26. The active compound chip as claimed in claim 25, **characterized in that** it contains Transfluthrin® or Pynamin forte® or benzyl benzoate as the active compound.

27. The active compound chip as claimed in one of claims 1 to 26, **characterized in that** the active compound or mixture of active compounds can be evaporated at a temperature of below 104 °C, preferably in the range of from 65 °C to 90 °C.

28. The active compound chip as claimed in one of claims 1 to 27, **characterized in that**, after a starting phase of 5 8-hour cycles, the amount of total formulation evaporated on average over a cycle contains at least 70%, preferably more than 90%, particularly preferably more than 99% of the active compound or mixture of active compounds.

29. A process for the production of an active compound chip as claimed in one of claims 1 to 28, **characterized in that** at least one heating element (23) is sprayed onto an endless metal tape, the individual heating elements (23) are punched out of the endless metal tape, the contacts (13, 14) are separated and the heating elements (23) are enclosed in a housing, the active compound or mixture of active compounds is added to the at least one heating element (23) in the housing (33, 35) and the housing is sealed.

30. A process for the production of an active compound chip as claimed in one of claims 1 to 28, **characterized in that** at least, one heating element (23) is sprayed onto an endless metal tape, the individual heating elements (23) are punched out of the endless metal tape, the contacts (13, 14) are separated and enclosed in a housing (33, 35) containing the active compound or mixture of active compounds (34) and the housing is sealed.

31. A process for the production of an active compound chip having a heating element (23) consisting of two strips (13, 14) as claimed in claim 20, **characterized in that** each strip (13, 14) is sprayed onto two endless metal tapes (31, 32), one endless metal tape (31) is cut between each two strips, the other endless metal tape (32) is cut between each strip and the heating elements (23) produced in this manner are enclosed in a housing (33, 35) which contains the active compound or mixture of active compounds (34) and the housing is sealed.

32. A process for the production of an active compound chip having a heating element (23) consisting of two strips (13, 14) as claimed in claim 31, **characterized in that** the first metal tape (31) is cut between each strip and an LED (15) is soldered in between each two strips.

33. A process for the production of an active compound chip, in which the active compound is bound to an active compound carrier, as claimed in one of claims 6 to 9, **characterized in that** a heating element (23) is sprayed onto at least one endless metal tape, the active compound carrier is sprayed around the heating element on the endless metal tape and the individual active compound chips are then punched out of the tape of active compound carriers on the endless metal tape and the contacts are separated.

34. A process for the production of an active compound chip, in which the active compound is bound to an active compound carrier, as claimed in one of claims 6 to 9, **characterized in that** the active compound carrier is a polymer and the heating element is a conductive plastic and the heating element together with the active compound carrier is extruded such that a plastic wire is produced whose core forms the heating element and whose jacket forms the active compound carrier.

## Revendications

1. Puce à matière active (1) avec au moins un élément chauffant (2) intégré, consistant en un matériau conducteur, usinable par machine et en deux contacts électriques (3, 4), depuis laquelle la matière active peut être vaporisée par chauffage de la puce (1) par mise d'une tension électrique aux contacts électriques (3, 4),
**caractérisée en ce que** l'élément chauffant (2) est incorporé dans la matière active ou dans un mélange de matières actives, le cas échéant en combinaison avec un support de matière active, de telle sorte que l'ensemble forme une unité.

2. Puce à matière active selon la revendication 1, **caractérisée en ce qu'**elle forme une plaque rectangulaire et présente une longueur dans l'intervalle de 10 à 100 mm, une largeur dans l'intervalle de 5 à 100 mm et une épaisseur dans l'intervalle de 3 à 20 mm.

3. Puce à matière active selon la revendication 1 ou 2, **caractérisée en ce que** l'élément chauffant est enfermé sur toute sa longueur dans la partie de la puce contenant la matière active, de telle sorte que la forme de l'élément chauffant se dessine sur la forme extérieure de la puce à matière active.

4. Puce à matière active selon l'une des revendications 1 à 3, **caractérisée en ce que** la matière active ou le mélange de matières actives se présente sous forme liquide ou solide et est doté d'une couche superficielle imperméable à la matière active ou au mélange de matières actives sous forme liquide ou solide et perméable à ceux-ci sous forme gazeuse.

5. Puce à matière active selon l'une des revendications 1 à 4, **caractérisée en ce que** la matière active est liquide, sous forme de gel ou solide et se présente dans un boîtier, et **en ce que** l'élément chauffant est incorporé dans elle.

6. Puce à matière active selon l'une des revendications 1 à 5, **caractérisée en ce que** la matière active ou le mélange de matières actives est lié à un support de matière active.

7. Puce à matière active selon la revendication 6, **caractérisée en ce que** le support de matière active est un polymère.

8. Puce à matière active selon la revendication 6 ou 7, **caractérisée en ce que** la puce consiste essentiellement en l'élément chauffant et en le support de matière active, avec la matière active ou le mélange de matières actives, qui entoure ce dernier.

9. Puce à matière active selon l'une des revendications 6 à 8, **caractérisée en ce que** le support de matière active consiste en des matières plastiques telles le polyéthylène, le polypropylène, les types TPX®, le Desmopan® 8410, le Vestamid® 1800, le BAK® 402-005 et **en ce que** la matière active consiste en Transfluthrin®.

10. Puce à matière active selon l'une des revendications 1 à 9, **caractérisée en ce que** l'élément chauffant consiste en céramique, en un conducteur de chauffage, en une feuille métallisée ou en un plastique conducteur.

11. Puce à matière active selon la revendication 10, **caractérisée en ce que** l'élément chauffant consiste en une résistance chauffante ou en une thermistance à CTP.

12. Puce à matière active selon l'une des revendications 1 à 11, **caractérisée en ce que** l'élément chauffant présente une résistance de 10 kΩ à 100 kΩ.

13. Puce à matière active selon l'une des revendications 1 à 11, **caractérisée en ce que** l'élément chauffant présente une résistance de 2 kΩ à 30 kΩ.

14. Puce à matière active selon l'une des revendications 1 à 13, **caractérisée en ce que** la puissance de chauffage de l'élément chauffant est comprise entre 0,1 W et 5 W.

15. Puce à matière active selon l'une des revendications 1 à 14, **caractérisée en ce qu'**avec l'élément chauffant, on peut régler une température dans l'intervalle de 60 °C à 140 °C.

16. Puce à matière active selon l'une des revendications 1 à 15, **caractérisée en ce que** l'élément chauffant (2) est disposé en forme de méandre avec au moins un coude (21), sachant que les deux contacts électriques (3, 4) se trouvent aux deux extrémités du méandre.

17. Puce à matière active selon l'une des revendications 1 à 15, **caractérisée en ce que** l'élément chauffant (22) est disposé en forme de deux méandres avec au moins un coude (21), sachant qu'un contact électrique (3, 3' ; 4, 4') respectivement se trouve à chaque extrémité des deux méandres.

18. Puce à matière active selon la revendication 17, **caractérisée en ce que** les contacts électriques (3', 4') tournés l'un vers l'autre des deux méandres sont reliés électriquement de manière conductrice.

19. Puce à matière active selon l'une des revendications 1 à 18, **caractérisée en ce que** l'élément chauffant (23) a une structure en grille ou en nid d'abeille.

20. Puce à matière active selon la revendication 19, **caractérisée en ce que** l'élément chauffant (23) avec la structure en grille ou en nid d'abeille est exécuté en deux bandes (11, 12) qui sont contactées chacune à une extrémité et sont reliées électriquement l'une à l'autre à l'extrémité opposée au contact.

21. Puce à matière active selon l'une des revendications 1 à 20, **caractérisée en ce que** les contacts électriques consistent en tôle de laiton ou en cuivre.

22. Puce à matière active selon l'une des revendications 1 à 21, **caractérisée en ce qu'**un affichage de fonctionnement (15) est intégré dans la puce.

23. Puce à matière active selon la revendication 22, **caractérisée en ce que** l'affichage de fonctionnement (15) est une LED bipolaire.

24. Puce à matière active selon l'une des revendications 1 à 23, **caractérisée en ce qu'**entre l'alimentation en courant et le contact est intercalée une puce de minuterie connue en soi et **en ce qu'**une résistance supplémentaire est intégrée dans la puce à matière active.

25. Puce à matière active selon l'une des revendications 1 à 24, **caractérisée en ce qu'**elle contient comme matières actives des pyréthroïdes, des matières actives acarides, des parfums ou des huiles essentielles.

26. Puce à matière active selon la revendication 25, **caractérisée en ce qu'**elle contient comme matière active du Transfluthrin® ou du Pynamin forte® ou du benzoate de benzyle.

27. Puce à matière active selon l'une des revendications 1 à 26, **caractérisée en ce que** la matière active ou le mélange de matières actives peut être évaporé à une température de moins de 100 °C, de préférence dans l'intervalle de 65 °C à 90 °C.

28. Puce à matière active selon l'une des revendications 1 à 27, **caractérisée en ce qu'**après une phase initiale de 5 cycles de 8 heures, la quantité de la formulation totale évaporée dans un cycle contient au moins 70 %, de préférence plus de 90 %, de façon particulièrement préférée plus de 99 % de la matière active ou du mélange de matières actives.

29. Procédé de fabrication d'une puce à matière active selon l'une des revendications 1 à 28, **caractérisé en ce qu'**au moins un élément chauffant (23) est pulvérisé sur un ruban métallique sans fin, **en ce que** les différents éléments chauffants (23) sont estampés depuis le ruban sans fin, **en ce que** les contacts (13, 14) sont sectionnés et **en ce que** l'on place les éléments chauffants (23) dans un boîtier, **en ce que** l'on ajoute la matière active ou le mélange de matières actives (34) à l'au moins un élément chauffant (23) dans le boîtier (33, 35) et **en ce que** l'on ferme le boîtier.

30. Procédé de fabrication d'une puce à matière active selon l'une des revendications 1 à 28, **caractérisé en ce qu'**au moins un élément chauffant (23) est pulvérisé sur un ruban métallique sans fin, **en ce que** les différents éléments chauffants (23) sont estampés depuis le ruban sans fin, **en ce que** les contacts (13, 14) sont sectionnés, et **en ce qu'**on les place dans un boîtier (33, 35) contenant la matière active ou le mélange de matières actives (34) et **en ce que** l'on ferme le boîtier.

31. Procédé de fabrication d'une puce à matière active avec un élément chauffant (23) qui consiste en deux bandes (13, 14) d'après la revendication 20, **caractérisé en ce que** chaque bande (13, 14) est pulvérisée sur deux rubans métalliques sans fin (31, 32), **en ce que** l'un ruban métallique sans fin (31) est sectionné entre deux bandes respectivement, **en ce que** l'autre ruban métallique sans fin (32) est sectionné entre chaque bande et **en ce que** les éléments chauffants (23) fabriqués de cette façon sont placés dans un boîtier (33, 35) qui contient la matière active ou le mélange de matières actives (34) et **en ce que** l'on ferme le boîtier.

32. Procédé de fabrication d'une puce à matière active avec un élément chauffant (23) qui consiste en deux bandes (13, 14) d'après la revendication 31, **caractérisé en ce que** le premier ruban métallique (31) est sectionné entre chaque bande et **en ce qu'**une LED (15) est brasée entre deux bandes respectivement.

33. Procédé de fabrication d'une puce à matière active, dans laquelle la matière active est liée à un support de matière active, selon l'une des revendications 6 à 9, **caractérisé en ce qu'**un élément chauffant (23) est pulvérisé sur au moins un ruban métallique sans fin, **en ce que** le support de matière active est pulvérisé autour de l'élément chauffant sur le ruban métallique sans fin et **en ce qu'**ensuite les différentes puces de matière active sont estampées depuis le ruban de supports de matière active sur le ruban métallique sans fin et **en ce que** les contacts sont sectionnés.

34. Procédé de fabrication d'une puce à matière active, dans laquelle la matière active est liée à un support de matière active, selon l'une des revendications 6 à 9, **caractérisé en ce que** le support de matière active est un polymère et l'élément chauffant est une matière plastique conductrice, et **en ce que** l'élément chauffant est extrudé ensemble avec le support de matière active, de telle sorte qu'on obtient un fil de plastique dont l'âme est formée par l'élément chauffant et l'enveloppe est formée par le support de matière active.
